# EUROPEAN PATENT SPECIFICATION

(11) **EP 2 712 378 B1**
(45) Date of publication and mention of the grant of the patent: **11.11.2020**
(21) Application number: 11862186.1
(22) Date of filing: 08.12.2011
(51) Int. Cl.: E04H 1/12, H04N 7/15

(54) **MEDICAL KIOSK AND METHOD OF USE**
MEDIZINISCHER KIOSK UND ANWENDUNGSVERFAHREN
KIOSQUE MÉDICAL ET PROCÉDÉ D'UTILISATION

(30) Priority: 31.03.2011 US 201161469851 P; 30.09.2011 US 201161541719 P
(43) Date of publication of application: 02.04.2014
(73) Proprietor: Rite Aid Hdqtrs. Corp., Camp Hill, PA 17011 (US)
(72) Inventor: CASHMAN, Steve, Powell, OH (US); SPIRK, John W., Gates Mills, OH 44040 (US); TILK, Jason G., Cleveland Heights, OH 44118 (US); NOTTINGHAM, John R., Bratenahl, OH 44108 (US); KALMAN, Jeffrey, Dublin, OH 43017 (US)
(74) Representative: Grosse Schumacher Knauer von Hirschhausen
(86) International application number: PCT/US2011/063895
(87) International publication number: WO 2012/134556

(56) References cited:
- DE-A1- 10 129 835
- US-A- 3 838 545
- US-A1- 2009 143 652
- US-A1- 2009 167 838
- US-A1- 2009 167 838
- US-A1- 2010 332 250
- US-A1- 2010 332 250
- US-B1- 6 205 716
- US-B1- 6 205 716

## Description

The present invention claims priority on United States Patent Application Serial Nos. 61/469,851 file March 31, 2011 and 61/541,719 filed September 30, 2011. The present invention also is a continuation-in-part of United States Design Patent Application Serial No. 29/403,857 filed October 12, 2011.

The present invention is directed to medical services, more particularly to a device for providing medical services to individuals, even more particularly to a device for providing medical services to individuals at locations that traditionally have not provided medical services, and still even more particularly to a medical kiosk for providing medical services to individuals at locations that are remote from a medical provider.

### BACKGROUND OF THE INVENTION

Medical services are traditionally provided to individuals at a doctor's office or medical facility. Typically, an individual contacts his/her medical provider when the individual requires some type of medical assistance. The medical provider then sets an appointment time and date for the individual to see the medical provider. Many times, the time and date of the appointment are inconvenient for the individual. Furthermore, the individual seeking medical assistance may desire or need more immediate medical assistance and cannot wait for the time and date set by the medical provider. In such situations, the individual goes to the emergency room of a hospital or some type of medical clinic (e.g., Minute Clinic, Take Care Clinic, Urgent Care Clinic, etc.), assuming that such clinics are available or convenient to visit.

The costs associated with visiting a medical provider can be costly depending on the type of insurance, if any, the individual carries. When an individual visits the emergency room of a hospital, the medical costs can be substantially higher and insurance coverage may be limited to various types of visits. Insurance coverage and cost of the visit may also vary at various clinics. In many communities, clinics are not readily available, thus the individual must either visit the medical provider or go to the hospital.
Various pharmacy and drug stores have begun offering medical services on their premises. These locations generally offer flu shots and very basic medical services, and are typically provided by a nurse practitioner, not a doctor. As such, only very limited types of medical services are offered at such locations. Also, these locations are not offered in a private environment. Generally, the services are provided in a side corridor or partitioned location in the facility.

In view of the current state of the medical services, there is a need for providing medical services in a more convenient, desirable, timely and cost effective manner. US 6,205,716 B1 discloses a modular video conference system according to the preamble of claim 1. US 2009/0167838 A1 discloses an apparatus and a method for cleaning a telemedicine booth.

### SUMMARY OF THE INVENTION

The present invention is directed to a device for providing medical services, diagnoses, health, and/or wellness advice to individuals in a convenient, desirable, timely and cost effective manner. The novel medical device of the present invention addresses the current deficiencies that exist for providing medical services to individuals.

In one non-limiting aspect of the invention, there is provided a remote medical service arrangement wherein a patient can receive various types of medical advice and services remotely from a medical provider (e.g., doctor, nurse practitioner, nurse, psychologist, optometrist, physician assistance, pharmacist, health coach, etc.). Traditionally, a patient was required to go to a medical facility (e.g., hospital, medical clinic, doctor's office, etc.) to personally meet with and be diagnosed by the medical provider. The present invention is directed to a device wherein medical services, diagnoses, health advice, and/or wellness advice can be dispensed by a medical provider at a location that is remote from the patient. In one non-limiting arrangement, there is provided an audio and/or video link between one or more medical providers located at one or more locations (e.g., medical provider's office, medical provider's home, hospital, etc.) and the patient is located at some other location (e.g., shopping mall, shopping center, drug store, grocery store, department store, warehouse store, discount retailer, discount department store, truck trailer, mobile office, mobile home, office space location, etc.) that is remote from the one or more medical providers. The novel device for providing medical services, diagnoses, health advice, and/or wellness advice enables a medical provider to provide medical services, diagnoses, health advice, and/or wellness advice to patients without the patient having to physically visit the medical provider and/or having to go to the medical provider's office or place of work. As can be appreciated, the audio and/or video link that is used by the medical provider can enable the medical provider to provide medical services, diagnoses, health advice, and/or wellness advice at a single remote location or a plurality of remote locations. When the audio and/or video link enables the medical provider to provide medical services, diagnoses, health advice, and/or wellness advice to a plurality of remote locations, a single medical provider and/or a plurality of medical providers can be used to provide medical services, diagnoses, health advice, and/or wellness advice to patients that are located at a variety of different remote locations. When a plurality of medical providers is used, the medical providers can be located at the same or different locations. As can be appreciated, the novel device for providing medical services allows for more flexibility for a patient to obtain medical services, diagnoses, health advice, and/or wellness advice. The site at which the patient obtains the medical services, diagnoses, health advice, and/or wellness advice can be located in non-traditional locations that are more convenient to a patient and/or can be located at multiple locations to provide easier and/or more convenient access to such medical services, diagnoses, health advice, and/or wellness advice. The multiple locations can be located in a single neighborhood, multiple neighborhoods, a single town or city, multiple towns or cities, a single state or province, multiple states or provinces, a single country, or multiple countries. The novel device can also be used to provide medical services, diagnoses, health advice, and/or wellness advice at non-standard hours (e.g., evening hours, weekend hours, holiday hours, etc.) to enable a patient to obtain medical services, diagnoses, health advice, and/or wellness advice that are more convenient and timely to the patient.

In another and/or alternative non-limiting aspect of the invention, the novel device of the present invention can be used to provide a variety of different medical services, diagnoses, health advice, and/or wellness advice. Non-limiting examples of such medical services, diagnoses, health advise, wellness advise and/or medical conditions that can be identified, treated and/or addressed include, but are not limited to Acid Reflux, Hypertension Management, Allergies, Athlete's Foot, Acne, Mental Health Counseling, Wellness Counseling, Asthma, Cold Sores, Vaccinations, Arthritis, Bronchitis, Impetigo, Wellness Coaching, Weight Loss, Eating Disorders, Bladder Infections, Insect Stings, Allergic Reactions, Hemorrhoids, Minor Burns, Health Risk Management, Migraine Headaches, Common Colds, Minor Skin Infections, Chronic Disease Management, Coughs, Poison Oak/Ivy, Diarrhea, Rashes, Diabetes, Ringworm, Lice, Ear Infections, Sties, Flu, Fever, Gout, Headache (minor), Pink Eye, Sinus Infections, Sore Throat, Ear Infections, Cramps, STDs, Strep Throat, Throat Infections, Feeding Problems For Newborns, Vomiting, Teething, Gastrointestinal Problems, Anxiety, Depression, Formula Advice For Newborns, Concussion, Head Injuries, Bone Fractures, Hair Loss, Alopecia, Eye Infections, Urinary Tract Infections, Constipation, Appendicitis, Pharyngitis, Medication Therapy Management etc. As can be appreciated, other or additional types of medical services and/or health care services can be provided. The medical services and/or health care services that can be provided can include, but are not limited to, 1) providing advice and/or recommendations about a medical condition, 2) diagnosing a medical condition, 3) providing referral services for a medical condition, 4) prescribing medicine for a medical condition, 5) periodically monitoring a medical condition, 6) providing routine check-up services, 7) providing advice and/or recommendations about medical and/or health matters, 8) providing a course of treatment for a medical condition, 8) providing health counseling, 9) providing health information, 10) providing wellness counseling, and/or 11) providing wellness information. As can be appreciated, other or additional services can be provided to the patient. In essence, any type of medical condition, medical concern, health concern, wellness concern, etc. can be addressed by the novel method for providing medical services of the present invention. Hereinafter, these services will be collectively referred to as medical services. As can be appreciated, the type of services provided to a patient will depend on the specific medical condition, medical concern, wellness concern, and/or health concern of the patient. In many instances, the medical provider will be able to diagnose, address and/or treat the specific medical condition, medical concern, wellness concern, health concern, etc. of the patient. In some instances, the specific medical condition, medical concern, wellness concern, health concern, etc. of the patient may be too complicated or complex to address via an audio and/or video link, thus the medical provider in such situations may have to refer the patient to a hospital, a doctor's office, counselor, psychiatrist/psychologist, medical specialist, health professional, dietician, rehabilitation facility, a traditional medical clinic for further counseling, treatment and/or diagnosis, or some other location or professional that can address the patient's needs and/or requirements.

In still another and/or alternative non-limiting aspect of the invention, the novel kiosk enables the patient to conveniently communicate with the medical provider. One or more medical kiosks can be used in the present invention. Generally, a plurality of medical kiosks that are located at one or more locations can be used; however, it can be appreciated that a single medical kiosk can be used in accordance with the present invention. Typically one or more medical providers provide services to one or more medical kiosks. The size, shape, configuration and look of the medical kiosk are non-limiting. In one non-limiting embodiment of the invention, the medical kiosk provides a private or semi-private environment for a patient to communicate with a medical provider that is located remotely from the medical kiosk. In one non-limiting arrangement, the medical kiosk includes an enclosure that is designed to enable a patient to enter the enclosure and communicate with the medical provider while in the enclosure. The size, shape and configuration of the enclosure of the medical kiosk are non-limiting. In another and/or alternative non-limiting arrangement, the medical kiosk includes one or more walls that form all or a portion of the enclosure. The enclosure may include one or more doors or entry points to enable a patient to enter and exit the enclosure. In another and/or alternative non-limiting arrangement, the medical kiosk includes a floor and/or a ceiling. The ceiling, when included, can include a portion that is partially or fully transparent; however, this is not required.

The medical kiosk is modular in configuration to enable the medical kiosk to be set up in various configurations to enable the medical kiosk to be used in various types of spaces. The medical kiosk can be formed of any number of materials (e.g., plastic, foam, metal, wood, etc.). The walls of the medical kiosk can be designed to be interchangeable to enable the door, when used, to be positioned on various locations on the medical kiosk; however, this is not required. The medical kiosk can include a floor and/or ceiling to provide increased privacy for the patient when the patient is inside the room or cavity of the medical kiosk; however, this is not required. The medical kiosk can include a table, ledge, bench, and/or seat on the interior and/or exterior of the medical kiosk; however, this is not required. Such table, ledge, bench, and/or seat, when used, can be designed to be connected in multiple locations on the exterior and/or interior of the medical kiosk; however, this is not required. The modular configuration of the medical kiosk can be such that it can be easily assembled and disassembled so that the medical kiosk can be easily brought into a location and easily set up, or be easily removed from a location; however, this is not required.

One or more data input terminals can be located on one or more locations on the exterior of the medical kiosk; however, this is not required. Alternatively or additionally, the one or more data input terminals can be located on one or more locations in the interior or enclosure of the medical kiosk; however, this is not required. The one or more data input terminals can include a video display to display information regarding identification and/or data entry, a camera and/or video camera used to collect information for identification and/or data entry, a key pad or key board for identification and/or data entry, a touch screen for identification and/or data entry, microphone and voice recognition software for identification and/or data entry, fingerprint scanner for identification and/or data entry, retina scanner for identification and/or data entry, and/or face and/or body scanners for identification and/or data entry. As can be appreciated, other or additional devices can be included on the medical kiosk to display and/or obtain information regarding identification and/or data entry. The medical kiosk can be used by the patient to enter/convey basic information about the patient. Such information includes, but is not limited to, a) patient name, b) patient address, c) patient contact information (e.g., home address, work address, phone number, email address, pager number, work number, etc.), d) patient age, e) patient sex, f) patient height, g) patient weight, h) patient medical history, i) current medicines used by patient, j) reason(s) for visit by patient, k) patient current symptoms, 1) patient insurance information, m) patient payment information, n) patient's current doctor, o) guardian or patent information, and/or p) next of kin information. As can be appreciated, other or additional information can be inputted/conveyed by the patient.

The kiosk can be designed to provide information to the patient prior to and/or during the inputting/conveying of information by the patient to the medical kiosk. In one non-limiting embodiment of the invention, the medical kiosk can include audio and/or visual instructions and/or displays used to provide a) information about the medical kiosk, b) how to use the medial kiosk, c) how to properly input/convey information to the medical kiosk, d) provide instructions and/or interactions with the patient during the inputting/conveying of information by the patient to the medical kiosk, e) the wait time for the patient's use of the medical kiosk, f) a list of patient's waiting to use the medical kiosk, and/or g) information regarding whether the medical kiosk is in use or is available. In another and/or alternative non-limiting embodiment of the invention, the medical kiosk can include light and/or sound indicators to provide information regarding whether the medical kiosk is in use or is available; however, this is not required. In still another and/or alternative non-limiting embodiment of the invention, the medical kiosk can include a notification system to a patient that the medical kiosk is available or will soon be available; however, this is not required. Such notification can be sent via email, phone, pager, internet, etc. Such notification system can be useful when the medical kiosk is not currently available to the patient. The patient can input the information into the medical kiosk and then go home, run other errands, etc., and then be later notified when the medical kiosk is available or will soon be available. The medical kiosk and/or notification system can also be used to inform the patient when and/or where other medical kiosks are available; however, this is not required. This service, when available, can be used to inform the patient that a nearby medical kiosk has a shorter wait period or is currently available, thus providing the patient with the option of traveling to another available medical kiosk instead of waiting for the current medical kiosk to become available; however, this is not required. This service, when available, can also be used to inform the patient when a prescription is ready for pickup and/or for conveying prescription information to the patient; however, this is not required. This service, when available, can also be used to inform the patient when a follow-up visit is due and/or scheduled; however, this is not required. As can be appreciated, the notification system can be used for other or additional services.

In yet another and/or alternative non-limiting aspect of the invention, one or more attendants can be used with the medical kiosk; however, this is not required. In one non-limiting embodiment of the invention, the medical kiosk can have one or more attendants assist a patient during the use of the medical kiosk (e.g., assist in check-in procedures, assist check-out procedures, assist in entering/exiting the medical kiosk, answering questions about the medical kiosk, assist in maintaining privacy/security of a patient while using the medical kiosk, assist user during payment of medical services, assist user in obtaining a prescription, etc.); however, this is not required. Such attendant, when used, can be a medical provider or non-medical provider. The one or more attendants, when used, can also or alternatively clean and/or sanitize various regions of the medical kiosk prior to and/or after being used by a patient and/or set up the medical kiosk for a new user; however, this is not required. For example, prior to and/or after one or more patients have entered the medical kiosk, the one or more attendants can clean/sanitize one or more exterior surfaces and/or regions of the medical kiosk (e.g., medical kiosk door, medical kiosk check-in terminal, medical kiosk desk top, medical kiosk exterior walls, medical kiosk touch screen, medical kiosk monitors, seating/tables in waiting area near medical kiosk, etc.); however, this is not required. In an another and/or additional example, prior to and/or after one or more patients have entered the medical kiosk, the one or more attendants can clean/sanitize one or more interior surfaces of the medical kiosk (e.g., medical kiosk door, medical kiosk floor, medical kiosk bench, medical kiosk chair, medical kiosk user terminal, medical kiosk interior desk top, medical kiosk interior walls, medical kiosk touch screen, medical kiosk monitors, medical kiosk instrument doors, medical devices/instruments used by and/or touched by user when in the medical kiosk, any other surface in the interior of the medical kiosk, etc.); however, this is not required. In still another and/or additional example, prior to and/or after one or more patients have entered the medical kiosk, the one or more attendants can set up the medical kiosk for a user (e.g., clean/sanitize interior surfaces of medical kiosk; clean/sanitize medical devices/instruments used and/or touched by a prior user; reposition medical devices/instruments into device storage areas; replace disposable components on medical devices/instruments; replenish paper in a printer; clear a paper jam in a printer; replace batteries for one or more electronic components; close medical device/instrument compartments doors in the medical kiosk; reset user touch screen for next user in the medical kiosk; fix, repair and/or replace non-operating, damaged or broken medical devices/instruments in the medical kiosk; fix, repair and/or replace electronic components, computers, fans, light bulbs, UV bulbs, UV devices, etc. in the interior and/or exterior of the medical kiosk; refill cleaning and/or sanitizing fluid; etc.); however, this is not required. In yet another and/or additional example, the one or more attendants can be used to assist one or more users in the medical kiosk. Generally, such assistance will occur only after requested by the user in the medical kiosk or by the medical provider that is assisting the user while in the medical kiosk; however, this is not required. For instance, the one or more attendants can assist a user in the medical kiosk if the one or more attendants hear a verbal request from the user, receive notice (e.g., light indicator activated by user, sound indicator activated by user, hear user talking via a speaker to attendant, hear user talking through walls of medical kiosk, receive a notice from the medical provider [e.g., phone call, email, light indicator, etc.], etc.); however, this is not required.

In still yet another and/or alternative non-limiting aspect of the invention, the medical kiosk can include a sanitizing system (e.g., UV system, mist system, etc.) that can be automatically activated and/or activated by the attendant prior to and/or after a patient has used the medical kiosk; however, this is not required.

In another and/or alternative non-limiting aspect of the invention, the medical kiosk can be made of one or more materials that resist or prevent the growth of bacteria, viruses and/or other micro-organisms; however, this is not required. In one non-limiting embodiment, the floor, walls and ceiling of the medical kiosk include or are fully made of materials that resist or prevent the growth of bacteria, viruses and/or other micro-organisms; however, this is not required.

The medical kiosk can include a payment center that enables a user to pay for medical services, prescriptions, medical equipment, medical accessories, etc. prior to and/or after the user uses the medical kiosk; however, this is not required. The payment center can be in any form (e.g., credit card reader, mobile phone scanner, transmitter/receiver device, electronic scanner, cash receiver, etc.). The payment center may include a touch pad, key board, scanner, receiver, transmitter, credit card/debit card or some other card reader, smart phone or other smart device reader/scanner, finger and/or eye scanner, monitor, chair, table, shelf, printer, instructions on how to use the payment center, etc. The payment center can be located on the exterior and/or interior of the medical kiosk. Generally, the user is required to register and pay for the medical services prior to obtaining medical services from the medical provider; however, this is not required. In one non-limiting arrangement, the medical kiosk includes a registration station on the exterior of the medical kiosk (e.g., exterior wall of the medical kiosk, on a table exterior to the medical kiosk, etc.); however, this is not required. As can be appreciated, a user can be allowed to wirelessly connect to the medical kiosk or to some other computer network so as to wirelessly register and/or enter payment information for use of the medical kiosk; however, this is not required. In such an arrangement, a user could register to use a medical kiosk, enter in payment for use of the medical kiosk, set an appointment time for use of the medical kiosk, select a particular medical kiosk to use at some particular location, etc. at some location near or remote from the medical kiosk via a smart phone or other smart device, a computer, etc.

The medical kiosk includes an interior cavity or room that provides privacy to the patient when inputting and/or conveying data to the medical kiosk, and/or communicating with a medical provider via an audio and/or video link. The size, configuration and/or arrangement of the interior cavity or room are non-limiting. The interior cavity or room can include a) one or more speakers, b) one or more microphones, c) one or more video displays, d) data input device, e) one or more chairs and/or other types of seating areas, f) one or more tables, g) one or more doors, h) one or more shelves, i) one or more compartments used to contain medical supplies, medical instruments, etc., j) one or more light switches, k) one or more power outlets, 1) sterilization system, and/or m) one or more lights. As can be appreciated, the interior cavity or room can include other or additional items. The size and configuration of the interior cavity or room can be designed to enable wheelchair access and maneuvering inside the interior cavity or room; however, this is not required. For example, the interior cavity or room can be designed to enable a standard wheelchair to move 90°, 180° and/or 360° while in the medical kiosk. The size and configuration of the interior cavity or room can be designed to provide sufficient room for the patient so that the patient can easily move within the interior cavity or room and/or the patient does not feel cramped or claustrophobic when in the interior cavity or room; however, this is not required.

In another and/or alternative non-limiting aspect of the invention, the novel method of the present invention, the method for providing medical services in accordance with the present invention is designed to provide a convenient and low-cost structure (e.g., medical kiosk) that can be placed in many different locations, and which enable patients to conveniently access and obtain medical advice and/or care. Medical providers that are located locally or throughout the world can be used to communicate with the patient accessing the medical kiosk. As such, the medical services can be offered year around and at all times so long as there is a qualified medical provider somewhere in the world that is available and is qualified to provide medical assistance via the medical kiosk. Such an arrangement can be more convenient to the medical provider since the medical provider can work from home or from some other convenient location. The arrangement is also convenient to the patient since the patient can access medical assistance via the medical kiosk at the time and place of choosing. Indeed, in rural areas or smaller communities that do not have a local hospital or local doctor's offices nearby, the installation of a medical kiosk in the local drug store, department store, grocery store, etc., results in more accessible and timely medical care for patients in such communities. The costs associated with providing medical care via the medical kiosk may be less than if the patient seeks medical assistance from a hospital, clinic or doctor's office, thus resulting in the patient potentially saving money. As can be appreciated, other or additional advantages may exist by the method of the present invention.

It is one non-limiting object of the invention to provide tele-med services that are convenient to a user.

It is another and/or alternative one non-limiting object of the invention to provide tele-med services that are cost effective to a user.

It is still another and/or alternative one non-limiting object of the invention to provide tele-med services that can be provided to a user via a medical kiosk.

It is yet another and/or alternative one non-limiting object of the invention to provide tele-med services that can be provided to a user via a modular medical kiosk.

It is still yet another and/or alternative one non-limiting object of the invention to provide a medical kiosk that is easy to assemble and disassemble.

It is another and/or alternative one non-limiting object of the invention to provide a medical kiosk that includes an easy and convenient registration system and payment system.

It is still another and/or alternative one non-limiting object of the invention to provide a medical kiosk that provides privacy to a user when obtaining medical services.

It is yet another and/or alternative one non-limiting object of the invention to provide a medical kiosk that sized and shaped to accommodate disabled or handicapped users.

It is still yet another and/or alternative one non-limiting object of the invention to provide a medical kiosk that includes medical instruments that can be used by a user when obtaining medical services.

It is another and/or alternative one non-limiting object of the invention to provide a medical kiosk that includes video conferencing capabilities between a user and a medical provider.

It is still another and/or alternative one non-limiting object of the invention to provide a medical kiosk that provides the option to play back one or more portions of the video conference session to the user after the video conference between the user and medical provider has been completed.

These and other objects and advantages will become apparent to those skilled in the art upon reading and following the description taken together with the accompanying drawings.

### BRIEF DESCRIPTION OF THE DRAWINGS

Reference may now be made to the drawings which illustrate various preferred embodiments that the invention may take in physical form and in certain parts and arrangement of parts wherein:
FIGURE 1 is an exploded view of a medical kiosk in accordance with the present invention;
FIGURE 2 is a front view of the medical kiosk of FIGURE 1;
FIGURE 3 is a top view of the medical kiosk of FIGURE 1;
FIGURE 4 is a bottom view of the medical kiosk of FIGURE 1;
FIGURE 5 is a first view of the medical kiosk of FIGURE 1;
FIGURE 6 is a second view of the medical kiosk of FIGURE 1;
FIGURE 7 is a back view of the medical kiosk of FIGURE 1;
FIGURE 8 is a top interior view of the medical kiosk of FIGURE 1;
FIGURE 9 is a cross-section view along line 9-9 of FIGURE 8;
FIGURE 10 is a cross-section view along line 10-10 of FIGURE 8;
FIGURE 11 is a cross-section view along line 10-10 of FIGURE 8 viewed from the opposite direction; and,

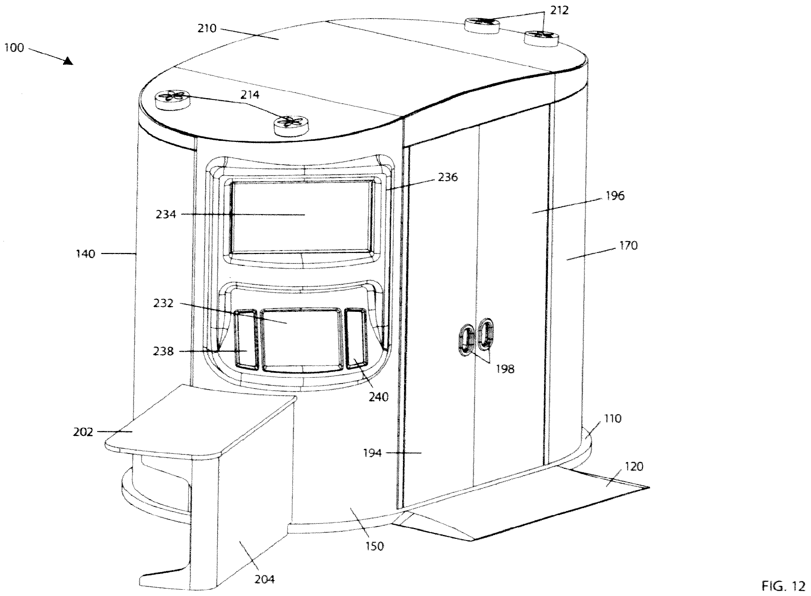

FIGURE 12 is a front-side elevation view of the medical kiosk of FIGURE 1.

### DETAILED DESCRIPTION OF ONE NON-LIMITING EMBODIMENT

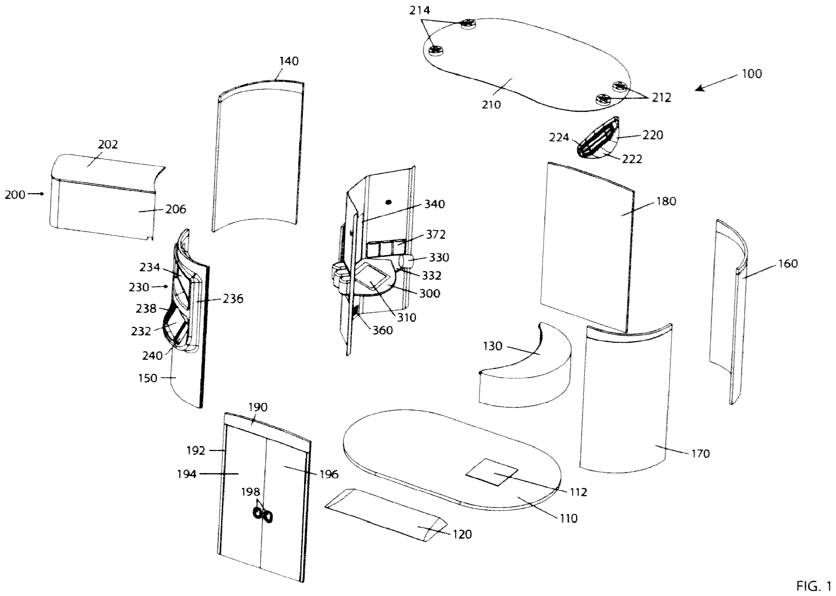

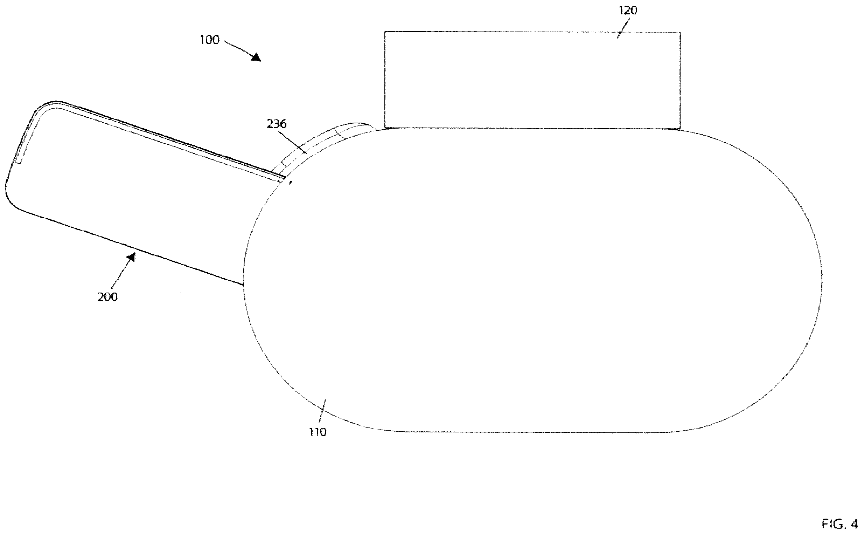

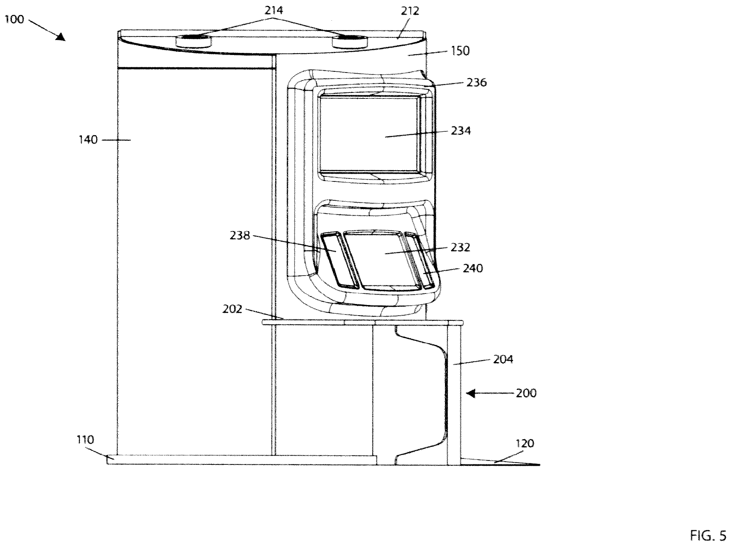

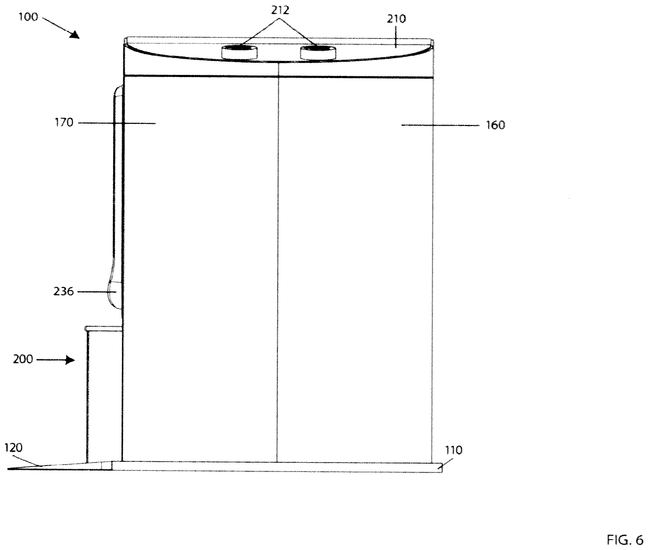

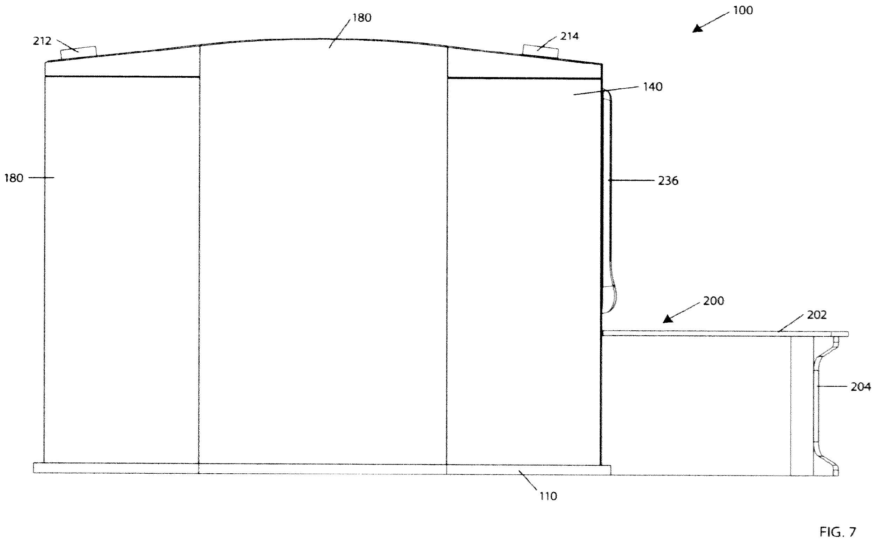

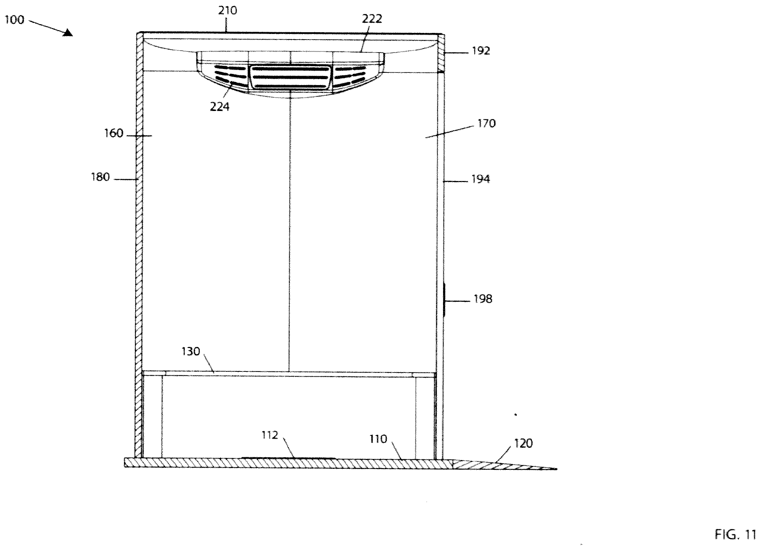

Referring now to the drawings wherein the showings are for the purpose of illustrating one non-limiting embodiment of the invention only and not for the purpose of limiting same, FIGURES 1-11 illustrate one non-limiting embodiment of the medical kiosk in accordance with the present invention. The medical kiosk 100 is designed to be used by a user to obtain medical services. Such medical services are generally tele-med services wherein one or more medical providers located at a remote location provide medical services to one or more users that are using the medical kiosk. As can be appreciated, non-tele-med services can also be provided to a user that is using the medical kiosk.

The shape, size and configuration of the medical kiosk are non-limiting. The materials and colors of the medical kiosk are also non-limiting. Generally, the materials used to form the medical kiosk include materials that resist or prevent microbial growth; however, this is not required. The medical kiosk illustrated in FIGURES 1-11 is designed to accommodate about 1-4 adults; however, it can be appreciated that the medical kiosk can be designed to accommodate additional users.

The medical kiosk is generally designed to be modular so that it can be easily assembled and disassembled; however, this is not required. FIGURE 1 illustrates one non-limiting set of modular components of the medical kiosk. The medical kiosk generally includes a floor panel 110; however, this is not required. The floor panel, when used, is generally a one piece unit; however, the floor panel can be formed of multiple pieces. The floor panel is generally formed of a durable material (e.g., plastic, metal, wood, composite material, man-made materials, etc.). The floor panel can be formed of a slightly compressible material to facilitate in the comfort of walking on the floor panel; however, this is not required. The floor panel is illustrated as having an oval shape; however, other shapes can be used (e.g., circular, square, rectangular, polygonal, etc.). The maximum length of the floor panel is generally 3-15 (0,9 - 4,5 m) feet, typically 4-12 feet (1,2 - 3,7 m), more typically about 6-10 feet (1,8 - 3,0 m), and even more typically about 8-9 feet (2,4 - 2,7 m); however, other lengths can be used. The maximum width of the floor panel is generally 3-10 feet (0,9 - 3,0m), typically 4-8 feet (1,2 - 2,4 m), and more typically about 4-6 feet (1,2 - 1,8 m); however; other widths can be used. The top surface area of the floor panel is generally 10-150 (0,9 - 13,9m²) sq. ft., typically 15-80 sq. ft. (1,4 - 7,4 m²), and more typically about 50-60 sq. ft. (4.7 / 5.6 m²); however, other surface areas of the floor panel can be used. The floor panel can be sized to enable a user in a wheelchair to enter the medical kiosk and turn and/or fully maneuver in the medical kiosk while sitting in the wheelchair; however, this is not required. The thickness of the floor panel is generally about 0.1-5 inches (0,3 - 12,7 cm), and typically about 0.25-3 inches (0,6 - 7,6 cm); however, other thicknesses of the floor panel can be used.

A weight scale 112 can optionally be partially or fully embedded in the floor panel. As can be appreciated, a weight scale can be placed on the top surface of the floor panel and/or positioned on other regions of the medical kiosk (e.g., chair, bench, etc.). The weight scale, when used, provides information about the weight of a user. The information from the scale can be electronically (e.g., wired, wirelessly) transferred to a medical provider and/or displayed to the user and/or medical provider.

A ramp 120 can be optionally used to facilitate entry and exiting of the medical kiosk; however, this is not required. The shape and size of the ramp are non-limiting. The ramp can be made of a similar material as the floor panel; however, this is not required. The ramp generally includes a sloped surface to facilitate in transitioning from a floor surface to the top surface of the floor panel; however, this is not required.

The medical kiosk can optionally include one or more benches 130, stools and/or chairs. When bench 130 is included in the medical kiosk, the bench is generally positioned on the back interior wall of the medical kiosk; however, this is not required. The bench can be used to allow a parent, guardian, spouse, relative, friend, etc. to sit in the medical kiosk while the user is obtaining medical services in the medical kiosk. The bench can be designed to enable one or more persons to sit on the bench. The bench can optionally include a storage space 132 under the seat of the bench that can be used to store supplies, equipment, etc. for the medical kiosk. A liftable seat section 134 can be used to access the storage space. When the bench includes a storage space, the bench can include a lock to limit access to the storage space; however, this is not required. As can be appreciated, the medical kiosk can include one or more chairs, not shown, to enable one or more users to sit in the medical kiosk while receiving medical services in the medical kiosk. The bench is generally about 10-25 (25 - 64 cm) inches high, and typically about 16-20 (41 - 51 cm) inches high; however, other heights can be used.

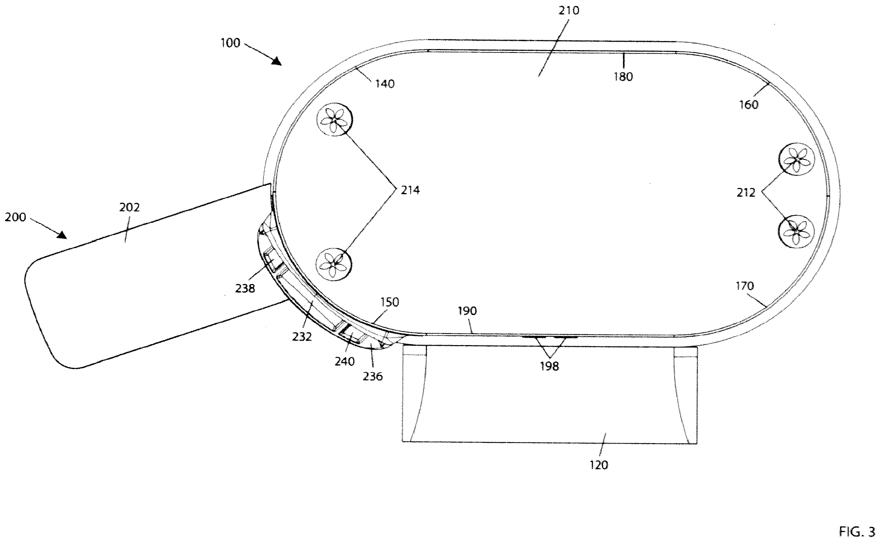

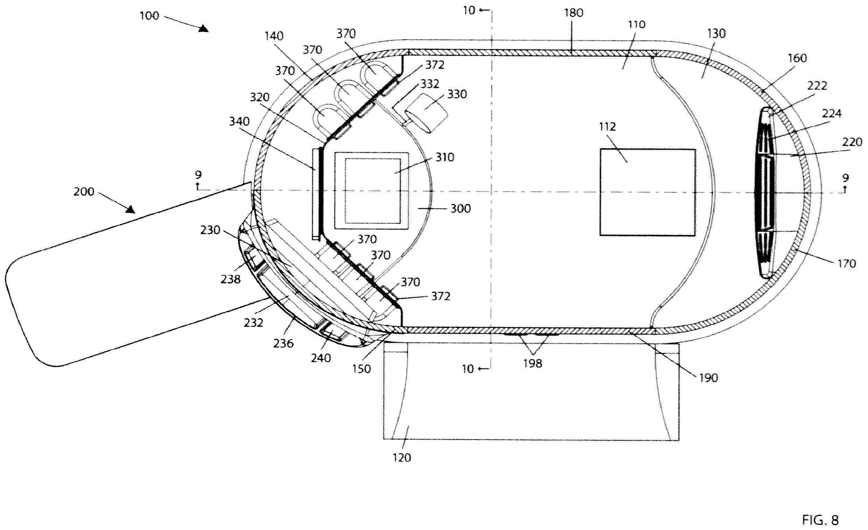

The medical kiosk is illustrated as having two front panels 140, 150, two rear panels 160, 170, one side wall 180, and one door system 190. The front panels, rear panels, side wall, and door system are generally formed of a durable material (e.g., plastic, metal, wood, composite material, man-made materials, etc.). As can be appreciated, the medical kiosk can be designed to only include a single front panel and/or a single rear panel. As can also be appreciated, the medical kiosk can be designed to include more than two front panels and/or more than two rear panels. As can be appreciated, the medical kiosk can be designed to include more than one side wall and/or more than one door system. As can also be appreciated, a side wall can be substituted for another door system; however, this is not required. The general shape and size of the front and rear panels are the same; however, this is not required. As illustrated in FIGURE 1, the shape of the front and rear panels is arcuate. The radius of curvature is about 10-100 inches, typically 15-50 inches (38 - 127 cm), and more typically about 20-35 inches (51 - 89 cm); however, other radius of curvatures can be used. As illustrated in FIGURES 1 and 3, the front and rear panels have an angle of curvature of about 90° or a quarter of a circle; however, it can be appreciated that one or both rear and/or front panels can have different angles of curvature. The general shape and size of the side wall and the door system are generally the same; however, this is not required. As illustrated in FIGURES 1 and 3, the side wall and door system lie in a generally flat plane; however, this is not required. As illustrated in FIGURE 8, the assembly of the front and rear panels and the side wall and door system forms a generally oval shape for the medical kiosk. The two front panels and two rear panels are illustrated as having the same or similar footprint. Likewise, the side wall and the door system have the same or similar footprint. The similarity in the shape and footprint of the wall components of the medical kiosk enables the medical kiosk to be assembled in a manner that is convenient for the facility that will include the medical kiosk. For example, if the door system needs to be positioned on the left side of the medical kiosk, instead of the right side, the similarly shaped side wall and door system enables the medical kiosk to be assembled in such a manner. Also, if the registration station of the medical system needs to be placed on the left side or right side or on the rear end of the medical kiosk instead of the front end, the similarly shaped front and rear panels can be easily exchanged to create such configuration for the medical kiosk. The modular medical kiosk not only accommodates multiple configurations of the medical kiosk, it also facilities in enabling the medical kiosk to be moved into an existing facility and then assembling the medical kiosk in such facility without having to modifying the entry ways into or out of the facility. The thickness and height of the front panels, rear panels, side wall and door system are non-limiting. Generally, the maximum height of the front panels, rear panels, side wall and door system is about 5-12 ft. (1,5 - 3,7 m), typically about 6-9 ft. (1,8 - 2,7 m), and more typically about 7-8 ft. (2,1 - 2,4 m); however, other heights can be used. The thickness of the front panels, rear panels, side wall and door system is generally about 0.5-10 inches (1,2 - 25 cm), typically about 1-5 inches (2,5 cm - 12,7 cm), and more typically about 1-2 inches (2,5 - 5,0 cm); however, other thicknesses can be used. The front panels, rear panels, side wall and door system can optionally include insulation, sound dampening material, etc.; however, this is not required. The front panels, rear panels, side wall and door system can be designed to be connected together in a variety of ways (e.g., bolted/screwed together, latched together, snap fitted together, press fitted together, etc.). Generally, the arrangement is used to connect together the front panels, rear panels, side wall and door system is selected to enable easy connecting and disconnecting of the front panels, rear panels, side wall and door system from one another. One or more of the front panels, rear panels, side wall and door system can include openings, windows, transparent/semi-transparent regions that allow for ventilation, illumination, and/or viewing; however, this is not required. Generally, front panels, rear panels, side wall and door system are mostly or fully formed of opaque or non-transparent materials so as to ensure the privacy of the user in the medical kiosk; however, this is not required.

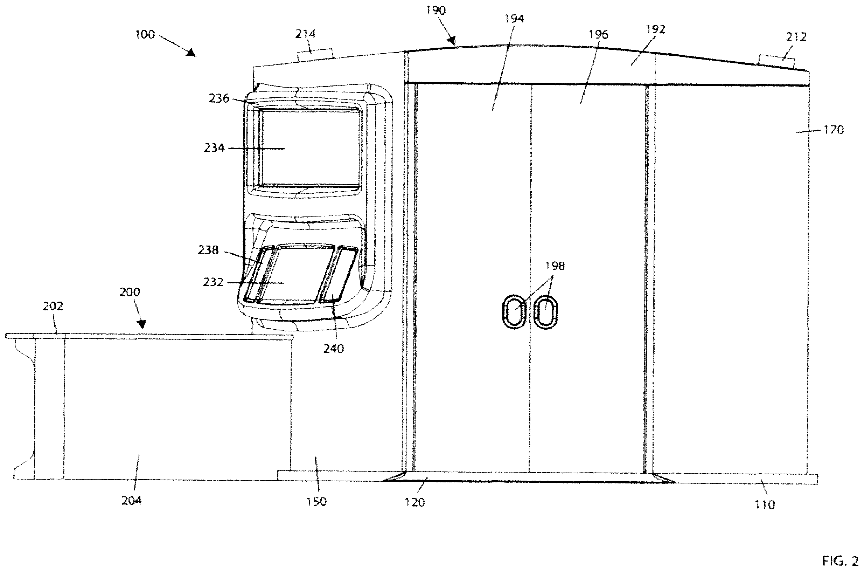

The configuration of the door system 190 is non-limiting. As illustrated in FIGURES 1 and 2, the door system includes a frame 192 and two doors 194, 196; however, it can be appreciated that the door system only includes a single door. Each door includes a handle or grasp cavity 198 on one or both sides of the one or both doors. The one or more doors can be designed to open and close in a variety of ways (e.g., swing open and closed, slide open and closed on a top/bottom rail system, etc.). As can be appreciated, the one or more doors for the medical kiosk can also or alternatively be positioned on one or more of the front or rear panels; however, this is not required. The maximum height of the doors is generally about 5-9 ft., and typically about 6-7 ft.; however, other heights can be used. The maximum width of the entry provided by the one or more doors when fully open is generally about 15-60 inches (38 - 152 cm), typically about 25-55 inches (64 - 140 cm), and more typically about 30-50 inches (76 - 127 cm); however, other widths can be used. The door opening is generally selected to enable a standard wheelchair to pass through the opening; however, this is not required. The door system can optionally include an indicator (e.g., light, message, etc.) that indicates when the medical kiosk is in use or is available. As can be appreciated, the use indicator can be located on other or additional locations on the medical kiosk. The door system can optionally include a lock arrangement. The configuration of the lock arrangement is non-limiting. The lock arrangement, when used, can be designed to enable the user to lock the doors to the kiosk medical to prevent access to the interior of the medical kiosk while the user is in the medical kiosk; however, this is not required. The lock arrangement can also or alternatively be used to lock and prevent access to the interior of the medical kiosk when the medical kiosk is not in use; however, this is not required.

The medical kiosk can optionally include an exterior attendant station that is connected to and/or positioned near the medical kiosk. The exterior attendant station can be used by one or more attendants, medical providers, etc. As illustrated in FIGURES 1-5, 7 and 8, a desk 200 can be connected to and/or positioned next to an exterior wall of the medical kiosk. The desk can be formed of one or more pieces. When the desk is designed to be connected to an exterior wall of the medical kiosk, such connection arrangement is not limited (e.g., screw, bolt, clamp, press fit, snap arrangement, etc.). The desk can include a desk top 202, one or more legs, one or more shelf regions 206, one or more drawers, etc. One or more chairs, not shown, can be used to allow one or more attendants, medical providers, etc. to sit at the desk. The one or more one or more attendants, medical providers, etc. located at the desk can assist a user in using the medical kiosk, maintain and/or clean the medical kiosk, provide medical services to a user of the medical kiosk, etc. The desk is illustrated as positioned at or adjacent to one or both front panels; however, this is not required. Generally the attendant, when used, is not a medical provider. The attendant, when used, is generally trained to assist a user to use the medical kiosk; however, this is not required. The attendant can 1) provide assistance to a user during the registration process and/or payment process, 2) provide assistance to a user about the medical kiosk and how to use the medical kiosk, 3) provide assistance to a user regarding technology in the medical kiosk and how to use such technology in the medical kiosk, 4) provide assistance to a user to enter and/or exit the medical kiosk, 5) clean and/or sanitize the medical kiosk, and/or 6) answer and/or assist the user in other ways regarding the medical kiosk and/or services provided by the medical kiosk.

The medical kiosk can optionally include a ceiling panel 210. The ceiling panel can be formed of one or more pieces. The ceiling panel can be formed of a transparent or semitransparent material to allow light to enter and illuminate the interior of the medical kiosk; however, this is not required. One or more lights, not shown, can be positioned on the ceiling panel to illuminate the interior of the medical kiosk; however, this is not required. The ceiling panel is illustrated as including four fan systems that are used to circulate air inside the medical kiosk. As can be appreciated, the location of the one or more fan systems on the ceiling panel is non-limiting. As can also be appreciated, more than or less than four fan systems can be positioned on the ceiling panel. As can also be appreciated, one or more fan systems can also or alternatively be positioned on other components of the medical kiosk (e.g., front panel, back panel, side wall, door system, floor panel, etc.). Generally, the rear fan systems 212 are designed to draw air into the medical kiosk and front fan systems 214 are designed to expel air out of the medical kiosk; however, this is not required. One or more fans systems can optionally include a filter system, to partially or fully filter dust, mites, airborne particles, micro-organisms, viruses, etc. from the air prior to the air entering into the medical kiosk and/or prior to the air exiting the medical kiosk; however, this is not required. The filter can include many different arrangements (e.g., HEPA filter, electronic filter, liquid filter, etc.).

The medical kiosk can optionally include a cleaning system that is designed to clean the interior of the medical kiosk and/or kill/neutralize some or all germs and/or other micro-organisms in the medical kiosk. One non-limiting cleaning system that can be used is a UV sanitizing system 220. As can also be appreciated, a mist sanitizer can also or alternatively be used to fully or partially clean/sanitize one or more portions of the medical kiosk. As illustrated in FIGURES 1, 8, 9 and 11, the UV sanitizing system 220 can be connected to or positioned adjacent to the ceiling panel and rear panels; however, this is not required. The UV sanitizing system generally includes one or more UV lights that are designed to kill some or all of the germs and/or other micro-organisms in the medical kiosk. Generally the germs and/or other micro-organisms in the medical kiosk are treated when the interior of the medical kiosk does not include a user. The UV sanitizing system can optionally include one or more standard lights that can be used to provide illumination in the medical kiosk; however, this is not required. The UV sanitizing system can optionally include one or more vents that allow air drawn into the medical kiosk by fan systems 212 to flow into the interior of the medical kiosk; however, this is not required. The UV sanitizing system can optionally include a cooling fan for the one or more UV lights and/or optional standard lights; however, this is not required. The UV sanitizing system can optionally include all or a portion of a mist sanitizing system; however, this is not required. The UV sanitizing system can house one or more cameras, speakers, sensors (e.g., temperature sensor, motion sensor, sound sensor, etc.), etc. for use in the medical kiosk; however, this is not required. The UV sanitizing system includes a shroud 222 that includes vent/light openings 224 to house the components of the UV sanitizing system. The shape, size and configuration of the shroud are non-limiting. When a mist sanitizing system is additionally or alternatively used, one or more mist nozzles can be located in one or more regions of the medical kiosk so as to direct the sanitizing mist to desired locations in the medical kiosk. The sanitizing system, when used, can be activated by an attendant and/or a medical provider while the medical kiosk is not being used by a user. The doors to the medical kiosk can be closed and/or locked to prevent a user from entering the medical kiosk during a sanitizing process; however, this is not required.

Referring now to FIGURES 1-3, 5 and 8, front panel 150 includes a registration station 230. The registration station is illustrated as including a touch screen 232, a display screen 234, and an optional frame 236 that such components can be mounted thereto. The shape of the frame, when used, is non-limiting. The frame, when used, can be designed to be easily removed from the front panel to enable servicing, repair, replacement, etc. of one or more components of the registration station; however, this is not required. As can be appreciated, the registration station can also or alternatively include other or optional features (e.g., additional display screen, additional touch screen, lights, buttons, switches, camera, speakers, microphone, keyboard, scanner, receiver, transmitter, credit card/debit card or other some other card reader, smart phone or other smart device reader/scanner, finger and/or eye scanner, shelf, printer, storage cavity, service access door, motion sensor, sound sensor, temperature sensor, logos, etc.). The touch screen is generally used to allow a user to enter in information about the user (e.g., age, sex, contact information, payment information, medical history, medical issue, etc.). The touch screen can be substituted for a keyboard; however, this not required. The frame is designed to mount the touch screen at some angle (e.g., 10-80) relative to the front plane of the front panel 150 as illustrated in FIGURES 1, 2 and 5; however, this is not required. The frame optionally includes one or more side sections 238, 240 that can include one or more other or optional features of the registration station. As can be appreciated, one or more other or optional features of the registration station can also or alternatively be located on other regions of the registration station. The touch screen can display various types of information (e.g., electronic keyboard, instructions on how to register, questions that are displayed during registration, instructions during registration, information displayed to user during registration, various templates, various menus, various lists of information, etc.). As can be appreciated, the medical kiosk can be designed to accept voice commands during the registration process; however, this is not required. The display screen can be used to provide various types of information (e.g., registration information, information input by the user, advertising information, information about the medical kiosk, information about wait time for a medical kiosk, information as to the order of users waiting to use the medical kiosk, information about whether a medical kiosk is available or in use, cable TV, satellite TV, local broadcast TV, infomercial, medical programs, DVD materials, Blu-ray materials, etc.). Generally, a user enters payment information at the registration station (e.g., swipes a credit or debit card, etc.); however, it can be appreciated that payment information can also or alternatively be entered inside the medical kiosk, at the optional attendant station, wirelessly or over a network via a smart phone or other device or by a computer connected to a network, etc. If an attendant is available, the attendant can assist a user during the registration process; however, this is not required. Generally, the medical kiosk includes a single registration station; however, this is not required. As can be appreciated, the registration station can alternatively be located inside the medical kiosk, at the attendant station, on other panels or sidewalls of the medical kiosk, or located remotely from the medical kiosk (e.g., central registration center for use with multiple medical kiosks, etc.).

Referring now to FIGURES 1 and 8-11, a non-limiting interior of the medical kiosk is illustrated. As previously discussed, the interior room or cavity of the medical kiosk can optionally include a scale 112, a bench 130 and/or a UV sanitizing system 220.

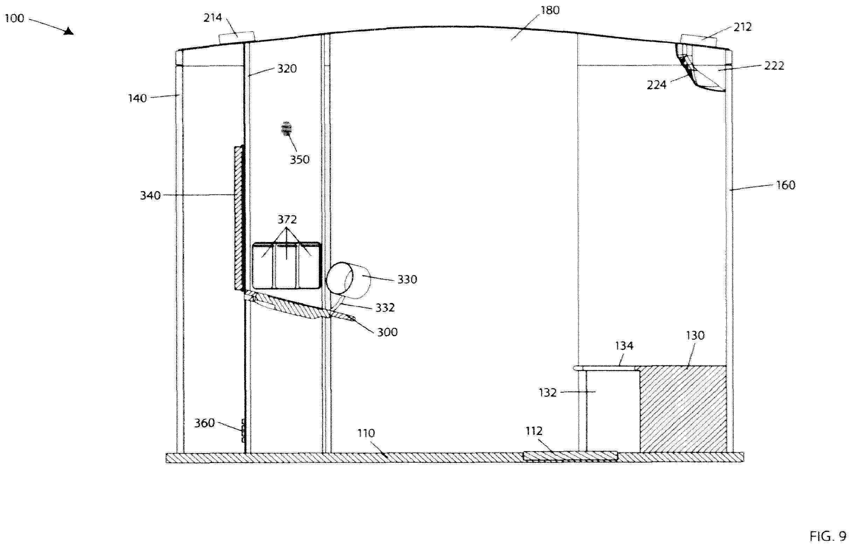

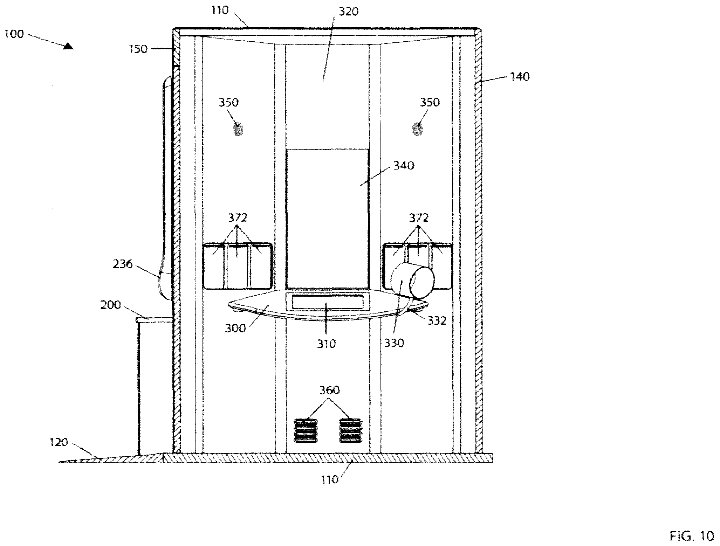

Referring now to FIGURES 9 and 10, a non-limiting interior front region of the medical kiosk is illustrated. A desk top 300 is used to support one or more touch pads 310 or keyboards positioned on the desk top. The shape, thickness and size of the desk top are non-limiting. The desk top is illustrated as secured to or formed on the front interior wall 320; however, this is not required. The desk top can have one or more support legs, not shown; however, this is not required. The one or more touch screens or keyboards on the desk top can be secured to the desk top; however, this is not required. The desk top is illustrated as sloping downward toward the front edge; however, this is not required. The size, shape and thickness of the one or more touch screens or keyboards are non-limiting. The one or more touch screens or keyboards are designed to be used by a user to enter various types of information (e.g., quality survey, patient history, patient medical information, payment information, questions to medical provider, etc.) before, during and/or after receiving medical services. The desk top can optionally include one or more other devices (lights, buttons, switches, camera, speakers, microphone, scanner, receiver, transmitter, credit card/debit card or other some other card reader, smart phone or other smart device reader/scanner, finger and/or eye scanner, shelf, printer, storage cavity, motion sensor, sound sensor, temperature sensor, logos, scanner, etc.); however, this is not required. A chair, not shown, can be provided to enable the user to sit when using the medical kiosk. The chair can be a free standing chair or be connected to the medical kiosk.

A blood pressure cuff 330 can be connected to the desk top; however, this is not required. The blood pressure cuff is illustrated as support by a cuff arm 332. The cuff arm can optionally be mounted to the desk top to enable the blood pressure cuff to be moved from side to side of the desk top; however, this is not required. Such movement of the blood pressure cuff enables the blood pressure cuff to be positioned so that the left or right arm of a user can be inserted into the blood pressure cuff when the user is facing the desk top. As can be appreciated, when a blood pressure cuff is included in the medical kiosk, it can be mounted and/or positioned in the medical kiosk in a variety of ways. Information to and from the blood pressure cuff, when used, can be transmitted by wire or wirelessly to one or more computers, processors, storage devices, etc. in the medical kiosk and/or to a location remote from the medical kiosk.

One or more monitors or display screens 340 can be positioned on the front interior wall 320. As can be appreciated, one or more monitors or display screens can be positioned on other or additional locations in the medical kiosk. The shape, size and thickness of the one or more monitors are non-limiting. The monitor is generally used to view the one or more medical providers when the user is located in the medical kiosk. The one or more monitors can also or alternatively display other or additional information (e.g., instructions, questions, general medical information, time, output or results of examination of user, advertisements, information about the medical kiosk, information being displayed and/or entered by the user on the touchscreen, etc.).

One or more cameras (e.g., video camera, etc.) can be positioned on the front interior wall and/or be embedded in the one or more monitors or display screens 340. As can be appreciated, one or more cameras can be positioned on other or additional locations in the medical kiosk. The one or more cameras enable pictures of the user in the medical kiosk to be transmitted to a remotely located medical provider. The remotely located medical provider also typically includes a camera at his/her location so that pictures of the medical provider can be transmitted to the one or more monitors or display screens 340 in the medical kiosk. Such an arrangement can allow for real-time or near real-time video conferencing between the user and medical provider while the user is located in the medical kiosk. The one or more cameras can have other or additional functions (e.g., determine height of user when standing on scale or other locations in the medical kiosk, view one or more regions of user so as to provide a medical examination of the user in the medical kiosk, monitor occupancy of the medical kiosk, provide security monitoring for the medical kiosk, etc.). The one or more monitors or display screens 340 in the medical kiosk can also be used to replay one or more portions of the video conference between the user and medical provider after the secession with the medical provider has ended. Such a feature enables the user to review or again listen to instruction, advice, etc. provided by the medical provider to the user. This video playback feature can be limited to being viewed by the user while the user remains in the medical kiosk, or can also or alternatively be accessed by the user after the user exits the medical kiosk. If the user is able to access the recorded video conference outside the medical kiosk, the video file can 1) be accessed from some central server, 2) electronically sent to a person computer, mobile device, tablet, laptop, etc., and/or 3) mailed to the user (e.g., DVD, Blu-ray disk, video tape, USB jump drive, etc.).

One or more speakers 350 can be positioned on the front interior wall of the medical kiosk. As can be appreciated, one or more speakers can be positioned on other or additional locations in the medical kiosk. The speakers can be used to enable a user in the medical kiosk to listen to what the medical provider is saying to the user. One or more microphones are generally included in the medical kiosk to enable the user to verbally communicate with the medical provider. The medical kiosk can optionally include a braille keyboard and/or reader to enable the visually and/or hearing impaired to communicate with a medical provider while in the medical kiosk. The speakers can also or alternatively be used to play background music, sound an alarm, etc.

The front interior wall can optionally include one or more air vents 360. The one or more air vents, when used, can enable air to enter or exit the interior of the medical kiosk to thereby provide circulation in the medical kiosk. As illustrated in FIGURES 8-10, the air flow into the medical kiosk is provided by fan systems 212. Fan systems 212 draw air into the medical kiosk and cause the air to flow through vents 224 in shroud 222 of the UV sanitizing system. The air continues to flow in the interior of the medical kiosk and into air vents 360. The air travels upwardly an interior wall space of the medical kiosk to provide cooling to monitors 340 and 234 and to the electronic equipment that is positioned in or near the inner space, and then out from the medical kiosk via fan systems 214. As can be appreciated, other or additional air flow paths can be created in the medical kiosk. As can be appreciated, one or more air vents can be located in other or additional regions of the medical kiosk.

One or more equipment chambers 370 can be positioned on or near the front interior wall. The equipment chambers are used to store one or more medical devices (e.g., stethoscope, otoscope, thermometer, dermascope, spirometer, pulse oximeter, heating pad, magnifying glass, tongue depressor, tweezers, blood glucometer etc.). The one or more equipment chambers can also or alternatively be used to include other types of materials (e.g., tissue, Band-Aid, gauze, cotton ball, disinfecting wipe, cortisone cream, anti-biotic cream/ointment, cotton swab, fabric wrap, etc.). The one or more equipment chambers generally include a door 372 to limit access to the one or more equipment chambers; however, this is not required. The door, when used, can be manually openable/closeable, and/or the doors can be controllably opened/closed remotely by the medical provider and/or attendant. Generally, the doors are controllably opened and/or unlocked by the medical provider during the examination of the user in the medical kiosk. After the user has left the medical kiosk, the attendant can enter the medical kiosk, and then clean the medical equipment that was handled or used by the prior user and/or dispose of and/or replace items that were used and/or handled by the prior user. Thereafter, the attendant can restock, replace, and/or reposition the medical equipment and/or non-medical equipment in the equipment chambers and close the equipment chamber doors prior to the next user entering the medical kiosk. One or more types of medical equipment can be designed to transmit information by wire or wirelessly to electronic components in the medical kiosk and/or to the remotely located medical provider. As illustrated in FIGURE 10, six equipment chambers having doors are included in the medical kiosk, three on each side of the desk top 300. As can be appreciated, a larger or smaller number of equipment chambers can be used. As also can be appreciated, some or all of the equipment chambers can be absent doors. The doors on the six equipment chambers are designed to be unlocked and/or opened remotely by the medical provider. The doors are designed to automatically lock closed when the doors are closed by the attendant after the user has left the medical kiosk; however, this is not required. Each of the six equipment chambers is designed to include a different piece of medical equipment, namely a stethoscope, an otoscope, a thermometer, a dermascope, a spirometer, and a pulse oximeter. As can be appreciated, a larger or smaller number of medical equipment can be used in the medical kiosk and/or different types of medical equipment can be included in the medical kiosk.

The medical kiosk and method for using the medical kiosk are a novel and advanced healthcare delivery system wherein patients and physicians can engage in real-time interactive consultations, providing convenient and affordable healthcare services. The medical kiosk includes the latest technologies in medical devices, video conferencing, and VOIP telephony so that the medical kiosk can extend traditional healthcare to convenient retail pharmacy locations or other locations in a user's neighborhood, therein enabling a user to see a medical provider and obtain a prescription, if required, in a fast and convenient manner.

Some advantageous aspect of the medical kiosk and medical method are:
- Patient Portal (Cloud Based).
- Doctor Portal (Cloud Based).
- Integrated Care Station.
- Facilitates Efficient Delivery of Basic Healthcare Delivery.
- Automates All Aspects of a Check Up.
- Easy Check-In.
- Vital Signs Capture.
- Prescription Generation.
- Post Care and Outcomes.
- Convenient Locations Where Consumers Want To Be.
- Video playback of the recorded session between the user and medical provider.

The medical kiosk and medical method can be used to provide primary and/or urgent care services in four (4) simple steps:
Step 1 - Patient requests appointment via web portal or walks to a medical kiosk and completes check in criteria.
Step 2 - Doctor receives eligible request and accepts.
Step 3 - Patient visits the medical kiosk and has a private appointment with a doctor via the doctor terminal.
Step 4 - Visit is completed. The medical kiosk and/or medical provider can then provide additional care/services that include: prescription, billing information, education, follow up and/or EMR/PHR entry. The medical provider can cause the medical kiosk to printout a prescription and/or directly send the prescription request to a pharmacy. The medical kiosk can print out a bill after the medical services are provided and/or accept payment prior to or after medical services are provided. The medical kiosk can be designed to accept and/or process medical insurance information provided by the user. The medical kiosk can print out and/or display education materials/information relevant to/requested by the user and/or provided by the medical provider. The medical kiosk and/or medical provider and/or attendant can schedule a follow-up visit for the user. Email, twitter, Facebook, phone and/or mail reminders can be sent to the user regarding scheduled and/or follow-up visits. The medical provider and/or attendant can schedule a visit with another medical provider and/or admit the user to the hospital, contact an ambulance, etc. during or after the visit to the medical kiosk.

Advantageous portal features of the medical kiosk and associated medical method are:
- Practice Management Engine.
   Appointments Scheduling Engine.
   Online Eligibility, Claims, and Billing Engine.
   ePrescribing with Alerts and Reminder Engine.
   Medical Records Interface and Access.
      Personal Health Record (PHR).
      Electronic Medical record (EMR).
      Rules-Based Care Plans.
      Rules-Based Education.
- Check In Pathway to Care Engine.
- Secure Video Conferencing Engine.
- Documentation Module.
   Appointment Storage and Analysis.
- Education and Post Care.

Some non-limiting advantages to patients by use of the medical kiosk and medical method are:
- Convenient.
   Closer to home.
   Saves time.
   Language and culture friendly.
- Better Access.
   Personal doctor available while traveling.
   Larger selection of doctors.
   Not limited by doctor's visitation schedule.
- More Accurate.
   Review record of appointment.
   Automatic data entry into PHR.
- Less Exposure to Illness.

Some non-limiting advantages to medical providers by use of the medical kiosk and medical method are:
- Higher Revenues.
   More appointments/day.
   Less traveling.
- More Accurate.
   Review record of appointment.
   Automatic data entry into EMR/HER.
- Integrated Care.
   Referral and transfer.
- Load Balancing.

Appointment load can be shared with other doctors regardless of location. Some non-limiting advantages to payers by use of the medical kiosk and medical method are:
- Change in Status.
   Transition from Payer to Provider.
- Market Leverage.
   New Business Model.
   Call Center based Nurse Practitioner.
- Efficiency.
   Market Demand.
   Less Overhead.
   Scalability.
   Less Liability.

The medical kiosk of the present invention is an Integrated Care Terminal that is a highly equipped doctor's office that is built and designed to deliver urgent and minor medical care in the field utilizing a centralized team of doctors for the evaluation and treatment of patients. The medical kiosk can be fitted with the latest FDA approved medical devices used by doctors today.

Employing the latest technology that is used in physician offices and emergency rooms, the medical kiosk is able to allow patients to obtain appropriate care in locations that are convenient, accessible, and more affordable.

The medical kiosk is designed to be a comfortable, self-contained, secure, sanitary, soundproof kiosk that is approximately 5 feet wide and 9 ½ feet long. The medical kiosk is made of extruded plastics and related components. The interior of the medical kiosk can contain one or more of the following integrated medical devices:
- Thermometer (e.g., temperature taken via ear, temperature taken via ear, IR thermometer to scan head or other area of body, etc.).
- Scale built into the patient seat or floor for measuring the patient's weight.
- Otoscope - for examining the middle ear, exterior ear, nasal passages, mouth and throat.
- Oximeter which measures the blood oxygen saturation.
- Stethoscope for evaluation of heart, lung and bowel sounds.
- Blood Pressure Cuff to measure blood pressure.
- EKG which provides a snapshot of the heart rhythm and data regarding stress or injury to the heart muscle.
- Spirometer and transducer for measuring lung function.
- Blood glucose measuring device or monitor.
- Retinal scan device (e.g., Itronix retinal scan device, etc.).

A nurse, nurse assistant, attendant, etc. who resides outside the medical kiosk can be responsible for answering user questions, assisting with user registration/payment, thoroughly clean the medical kiosk after each use, and restock and/or reset the medical kiosk after each use. The cleaning of the medical kiosk can include sanitizing the seat and all instruments as well as ensuring the medical kiosk is free of debris and any patient belongings. The medical kiosk can also be designed to be automatically sterilized after one or more users use the medical kiosk by utilizing a chemical mist sterilization technology and /or UV sterilization technology. The nurse, nurse assistant, attendant, etc. can also ensure that any insurance forms required by the user/patient for reimbursement are provided via a printer or some other means contained in the exterior and/or interior of the medical kiosk.

The medical kiosk can also contain a computer, which is connected to the internet and powers the one or more monitors and/or other type of equipment in the medical kiosk; however, this is not required. The exterior monitor on the medical kiosk can be used for patient registration and appointment selection that can be conducted in a touch screen format.

The method for providing medical services via a medical kiosk regarding protocols for scheduling, diagnosing, delivering and documenting telemedicine primary care can include:
a. Medical Provider web portal - this application is used by the medical provider to provide clinical services. The application contains all that the medical provider requires to diagnose, deliver care and document the clinical episode. It runs on the physician's computer and can be integrated with the leading EMR applications via HL/7, etc. One part of the medical provider portal is the telemedicine protocols and interview wizards which take the medical provider through a guided process for the clinical tele-services. [Robert - Steve Barrett questioned whether this section and following sections were correct]
b. Patient web portal - this application is used by the patient to register with a medical kiosk and also captures the patient's medical history. It includes all the information required to administer clinical services to the patient. This includes financial/billing information and a Healthspot Electronic Medical Record (EMR), which can be accessed by the patient and the medical providers.
c. Integrated videoconferencing software - this application supports the live patient-clinician interaction required for delivery of the clinical services. It uses a secure connection to the servers and the provider via an internet connection.

To use a medical kiosk, the users/patients may go through one or more of the following steps:
a. Go Online, register and schedule an appointment at the nearest terminal or walk-in and register at the medical kiosk.
b. Use our online scheduling engine to select an appointment time.
c. Input insurance, preferred pharmacy location and billing information, and remit payment.
d. Complete pre-appointment pathway to care.
e. Visit a medical kiosk and see a medical provider via the integrated care terminal.
f. Pick up prescription, if indicated, at the pharmacy of choice.
g. Use website to manage user's care until user is better.

The following example is a non-limiting example as to what one user may encounter when using the medical kiosk of the present invention:
Jane Doe is not feeling well, suffering from severe nasal congestion and "cold symptoms" for several days. She realizes she should seek care, but finds it frustrating to schedule an appointment with her primary care physician and knows that this is not an appropriate reason to visit her local ER. However, she recently became aware of a medical kiosk in her neighborhood grocery store, and decides to drive to the store a few minutes from her home to see if she can get a walk-in appointment. Upon arrival, Jane is met by the attendant who helps her register at the exterior of the medical kiosk for the next available appointment in a few minutes. She completes the basic information about her complaint in a short guided questionnaire via the monitor on the exterior of the terminal. The intake questionnaire captures demographic information include some or all of the following information: fingerprint, simple medical history/medications, current symptoms, preferred pharmacy, insurance information, and a fixed fee credit card payment for the visit. Information is gathered and stored securely to facilitate future visits. Jane then has a seat at a small waiting area near the medical kiosk. When the previous appointment ends by another user, Jane witnesses the attendant cleaning the appropriate surface areas of the interior of the medical kiosk, changing the protective covers of the instruments that were used, and hears a sterilizing mist sprayed into the medical kiosk.

Jane then swipes her fingerprint to verify her identity, enters the medical kiosk and is seated. Once her identity is verified, she is weighed by the scale and receives a friendly greeting from the live medical provider on the television monitor. The medical provider has already spent some time scanning Jane's medical history, current medications and symptoms. The medical provider then interviews Jane following the diagnostic telemedicine protocols, which are queued up, on the medical provider's home or office computer. Jane can see and speak to the medical provider and the medical provider can see and speak to Jane via the monitor facing Jane's chair.

After the medical provider has spoken to Jane and taken a medical history, he suspects a possible sinus infection. The medical provider then clicks on a button in the physician computer application, which creates a signal (e.g., green light, etc.) to open a door to a medical device cavity or compartment on the medical kiosk or turns on a light in the medical kiosk to identify the compartment that includes the otoscope. The medical provider than instructs Jane to gently insert the device in her nose and takes a picture of her nasal mucosa. The picture is then displayed on the side of the monitor where Jane can see it. The medical provider proceeds to instruct Jane to use the instrument to look in her ears and throat. The pictures are all queued on the side of the monitor. The medical provider then uses a tele-pen to highlight areas on the pictures of Jane's throat and nasal passages, which are inflamed and indicative of an upper respiratory infection. Based on her symptoms and the examination, the medical provider tells Jane he believes that she has a sinus infection.

Jane sees the pictures and feels comfortable with the diagnosis of a sinus infection, as she was able to witness firsthand the pictures with the telestrator. The medical provider thanks Jane for her visit and follows the protocols for wrapping up the visit. A prescription for the appropriate antibiotic is e-prescribed by the medical provider and sent electronically to the pharmacy of Jane's choice, which in this case happens to be the pharmacy within the grocery store. Upon exiting the terminal, Jane receives a completed Hgfa insurance form from the attendant, which she can use to file with her insurance company for reimbursement. Jane then walks over to the pharmacy and waits on her prescription, which is available ten minutes later. She returns home and begins taking the medication to cure her sinus infection.

Some of the non-limiting features of the medical kiosk are:
- Integrated Medical Devices.
- Exterior Check-In Station.
- Patient Waiting Area.
- Integrated Wi-Fi Hot Spot.
- Touch Screen User Interface.
- HIPAA Compliant Design.
- Instant Sterilization.
- Video/Audio Conferencing.
- Flexible Access.
   Handicap.
   Parent and Child.
- Modular design for Pharmacy door deployment.
- Small Footprint.
- Fully Integrated Interior Design.
- Expandable Device Rail.
- Secure PC storage with access.
- Open design feels comfortable.
- Payment and Signature.
- Finger Print Reader.
- Integrated Printer.
- Integrated Medical Devices.
   Thermometer - (e.g., temperature taken via ear, temperature taken via ear, IR thermometer to scan head or other area of body, etc.
   Scale built into the patient seat or floor for measuring the patient's weight.
   Otoscope - for examining the middle ear, exterior ear, nasal passages, mouth and throat.
   Oximeter which measures the blood oxygen saturation.
   Stethoscope for evaluation of heart, lung and bowel sounds.
   Blood Pressure Cuff to measure blood pressure.
   EKG which provides a snapshot of the heart rhythm and data regarding stress or injury to the heart muscle.
   Spirometer and transducer for measuring lung function.
   Blood Glucose measurement device and/or monitor to measure blood glucose levels.
   Retinal scan device to view structures in the eye.
- Exterior Check-In Station - A monitor and keyboard is generally mounted on the outside of the medical kiosk to allow for new user/patient registration and check in. The station can be designed to take payment and/or a fingerprint. The station generally is located away from the entrance to the medical kiosk to allow a degree of separation from the patient inside the medical kiosk. The attendant can use this station for her work.
- Patient Waiting Area - Can include a small area to put a few chairs outside the medical kiosk to act as a waiting area.
- Integrated Wi-Fi Hot Spot - In order to minimize network connection cost, a Wi-Fi hot spot can partner with an ISP of choice (AT&T, Verizon, Sprint, T-Mobile, etc.).
- Touch Screen User Interface - The patient can be allowed to interact while inside the medical kiosk by use of a touch screen interface.
- HIPAA Compliant Design - HIPAA requires patient information to be secure. This will mean the medical kiosk will generally be sound proof and a passerby cannot see in and see any patient information. The medical kiosk generally is fully enclosed and lockable, but can allow exterior access in case of emergency.
- Instant Sterilization - Because of the many germs and other contaminants that will be inside the medical kiosk, a sterilization technology can be used in the medical kiosk. One type of sterilization system that can be used is a built-in sanitizing misting system that dispenses from a series of misters between every appointment. Another or additional sterilization system that can be used is a UV lighting system that can be blasted between appointments. Other techniques and technologies can also or alternatively be used. The attendant can have the ability to activate one or more sterilization systems (e.g., via button, computer, etc.). The attendant can be required to keep track of records about the sanitization process and can ensure that the doors to the medical kiosk are closed/locked during the sterilization process.
- Video/Audio Conferencing - The patient will communicate with the medical provider via video and audio conferencing technology. This environment can make the patient feel as close to the medical provider as actually being present as possible. A two-way glass can be used to place the camera in the center of the monitor to keep the patient looking head on, versus the Skype and current video conferencing solutions that keep users looking at the camera and back to the monitor.
- Flexible Access - The medical kiosk should have a large enough door to accommodate a wheel chair. It should also be large enough inside to allow a parent to sit with a child and not feel constrained.
- Modular design for Pharmacy door deployment - Most Pharmacies today only have a standard door. The medical kiosk is built in a manner that allows it to enter through the door and quickly be assembled.
- Small Footprint - Because of the cost of retail space, the medical kiosk will be small enough to fit in most locations (e.g., 4-6 ft. wide and 7-10 ft. length).
- Fully Integrated Interior Design - The interior of the medical kiosk can be designed to be cleaner, sleeker, nicer, more luxurious, than the experience they get at the average doctor's office.
- Expandable Device Rail - The medical kiosk can include the latest medical devices and update such medical devices in a more rapid manner than the average doctors' office. The medical kiosk can include a mounting rail type system that allows medical devices in the medical kiosk to be easily accessed by the user. An indicator, such as a light, can be used to notify which medical device is to be used by the user.
- Secure PC storage with access - Because uptime of the software is so important, electronics can be inserted in a compartment in the medical kiosk. Such area generally should be secure, cooled, and easily accessible for service.
- Open design feels spacious - The medical kiosk is generally designed to feel bigger than it is such as by providing a glass window that wraps around the back half of the kiosk.
- Payment and Signature - The medical kiosk can include an integrated credit card swipe for payment, and a signature pad for medical authorization.
- Finger Print Reader - A finger print reader can be included on the medical kiosk to confirm patient ID under HIPAA.
- Integrated Printer - An integrated printer can be included in the medical kiosk to print medical and insurance forms and/or receipts and/or prescription. The printer can also print coupons based upon diagnosis to promote product sales. Our software can be included to inform the attendant of low paper in the paper.
- Video Playback - The recorded medical session can be partially or fully reviewed by the user to enable the user to again listen to information, instructions and/or advise from the medical provider. As can be appreciated, the video playback feature can also or alternatively be used for auditing purposes, compliance purposes, security purposes, quality control purposes, etc.

It will thus be seen that the objects set forth above, among those made apparent from the preceding description, are efficiently attained, and since certain changes may be made in the constructions set forth without departing from the spirit and scope of the invention, it is intended that all matter contained in the above description and shown in the accompanying drawings shall be interpreted as illustrative and not in a limiting sense. The invention has been described with reference to preferred and alternate embodiments. Modifications and alterations will become apparent to those skilled in the art upon reading and understanding the detailed discussion of the invention provided herein. This invention is intended to include all such modifications and alterations insofar as they come within the scope of the present invention. It is also to be understood that the following claims are intended to cover all of the generic and specific features of the invention herein described and all statements of the scope of the invention, which, as a matter of language, might be said to fall therebetween. The invention has been described with reference to the preferred embodiments. These and other modifications of the preferred embodiments as well as other embodiments of the invention will be obvious from the disclosure herein, whereby the foregoing descriptive matter is to be interpreted merely as illustrative of the invention and not as a limitation. It is intended to include all such modifications and alterations insofar as they come within the scope of the appended claims.

## Claims

1. A medical kiosk (100) designed to provide tele-med services to a user said medical kiosk having a kiosk structure that includes a front panel (140, 150), a plurality of medical devices and a user video conferencing system, said user video conferencing system designed to enable the user to have a real-time or near real-time tele-conference with a medical provider located remotely from said medical kiosk (100), said user video conferencing system includes a first display screen (340), information entry device, a camera, and a computer system and videoconferencing software, said first display screen configured to display said medical provider during a tele-conference between said user and said medical provider, said information entry device configured to enable said user to enter information, said first display screen (340) and said information entry device positioned in said kiosk such that said user can simultaneously view said first display screen (340) and said information entry device, said computer system and videoconferencing software including electronic hardware and software to form a video connection between said medical kiosk (100) and the remotely-located medical provider so that the remotely-located medical provider is displayed in real-time or near real-time on said first display screen, **characterized by** the medical kiosk having a modular design wherein an interior chamber is defined by one or more sidewalls, front panels, rear panels, and floor panels, and wherein said interior chamber is sized and configured to allow access within the interior chamber, and each of said one or more sidewalls, front panels, rear panels, and floor panels defines a length and a width capable of fitting through a standard door; and the interior chamber is provided with one or more medical compartments, wherein at least one of said compartments includes at least one medical device configured for use by the user to provide information to the medical provider about the user to enable the medical provider to provide real-time or near real-time medical advice to the user while the user is in said kiosk (100); and wherein the one or more medical compartment is a remotely controlled lockable medical compartment, wherein said medical provider can remotely and selectively lock or unlock the medical compartment.

2. The medical kiosk as defined in claim 1, wherein kiosk structure includes an interior chamber, said interior chamber includes said front panel, a rear panel and first and second side panels, said first display screen, said information entry device, said camera, and said plurality of medical devices located in said interior chamber, said interior chamber including an access door to enable a user to enter and to exit said interior chamber, said access door movable between an open and a closed position, said access door in said closed position providing privacy to said user when in said interior chamber.

3. The medical kiosk as defined in claim 1 or 2, wherein said front panel includes a desktop (202), said first display screen spaced above said desktop.

4. The medical kiosk as defined in any one of claims 1-3, wherein said information entry device is positioned on said desktop and below said first display screen.

5. The medical kiosk as defined in any one of claims 1-4, wherein said information entry device includes a keyboard or a touch screen.

6. The medical kiosk as defined in any one of claims 2-5, wherein said front panel optionally includes at least one of said medical compartments located on each side of said first display screen.

7. The medical kiosk as defined in claim 6, wherein at least one of said medical compartments is a lockable medical compartment, said lockable medical compartment configured to prevent access by said user to said medical device in said lockable medical compartment when a door to said lockable medical compartment is in a locked position, said remotely controlled lockable medical compartment includes a movable and lockable door that limits access to said at least one medical device in said remotely controlled lockable medical compartment, said door moveable between a closed position and an open position, said closed position of said door limiting access to said medical device in said remotely controlled lockable medical compartment, said open position providing access to said medical device in remotely controlled lockable medical compartment, said door on said remotely controlled lockable medical compartment is selectively unlockable by said medical provider to cause said door to move from said closed position to said open position.

8. The medical kiosk as defined in any one of claims 1-7, including a registration station positioned outside of said interior chamber, said registration station including a user input system that enables the user to enter information about the user prior to having said real-time or near real-time tele-conference with a medical provider, said user input system optionally including one or more components selected from the group consisting of a keypad for identification, keypad for data entry, keyboard for identification, keyboard for data entry, touch screen for identification, touch screen for data entry, microphone and voice recognition software for identification, microphone and voice recognition software for data entry, fingerprint scanner for identification, fingerprint scanner for data entry, retina scanner for identification, retina scanner for data entry, card reader, and smart device reader/scanner.

9. The medical kiosk as defined in any one of claims 1-8, including a floor panel, said medical kiosk including one or more accessories positioned at least partially on or in said floor panel, said accessories selected from the group consisting of a bench and a scale.

10. The medical kiosk as defined in any one of claims 1-9, including a cleaning or sanitizing system (220), to clean or sanitize at least a portion of said interior chamber, said cleaning or sanitizing system (220) including a system selected from the group consisting of a UV system and a mist system.

11. The medical kiosk as defined in any one of claims 1-10, including an attendant's desk (200) positioned outside said interior chamber.

12. The medical kiosk as defined any one of claims 1-11, including a ceiling panel (210) and a fan system (212) located on said ceiling panel (210), said fan system (212) designed to draw air into said interior chamber, draw air out of said interior chamber or combinations thereof, said fan system including a filtering system.

13. The medical kiosk as defined in any one of claims 1-12, wherein said camera is located in a position selected from the group consisting of said front panel (140, 150) or said first display screen (340).

14. The medical kiosk as defined in any one of claims 1-13, wherein said kiosk structure includes a printer.

15. The medical kiosk as defined in any one of claims 1-14, including a display screen located external to an internal enclosure of said medical kiosk that can be used to provide one or more types of information selected from the group consisting of advertising information, information about the medical kiosk, information about wait time for the medical kiosk, information as to the order of users waiting to use the medical kiosk, information about whether a medical kiosk is available or in use, cable TV shows, satellite TV shows, local broadcast TV shows, infomercials, medical programs, DVD materials, Blu-ray materials, display programs, You-Tube programs, and pictures, said display configured to only display information and to not accept data entry from the user.

## Patentansprüche

1. Medizinischer Kiosk (100), der zum Bereitstellen von telemedizinischen Dienstleistungen an einen Benutzer ausgelegt ist, wobei der medizinische Kiosk eine Kioskstruktur aufweist, die eine Frontplatte (140, 150), mehrere medizinische Geräte und ein Benutzer-Videokonferenzsystem beinhaltet, wobei das Benutzer-Videokonferenzsystem ausgelegt ist, dem Benutzer eine Echtzeit- oder Fast-Echtzeit-Telekonferenz mit einem medizinischen Dienstleister, der sich entfernt von dem medizinischen Kiosk (100) befindet, zu ermöglichen, wobei das Benutzer-Videokonferenzsystem einen ersten Anzeigebildschirm (340), ein Informationseingabegerät, eine Kamera und ein Computersystem und Videokonferenz-Software beinhaltet, wobei der erste Anzeigebildschirm zum Anzeigen des medizinischen Dienstleisters während einer Telekonferenz zwischen dem Benutzer und dem medizinischen Dienstleister konfiguriert ist, wobei das Informationseingabegerät konfiguriert ist, dem Benutzer das Eingeben von Informationen zu ermöglichen, wobei der erste Anzeigebildschirm (340) und das Informationseingabegerät derart in dem Kiosk positioniert sind, dass der Benutzer den ersten Anzeigebildschirm (340) und das Informationseingabegerät gleichzeitig sehen kann, wobei das Computersystem und die Videokonferenz-Software elektronische Hardware und Software zum Herstellen einer Videoverbindung zwischen dem medizinischen Kiosk (100) und dem entfernt befindlichen medizinischen Dienstleister beinhalten, sodass der entfernt befindliche medizinische Dienstleister in Echtzeit oder Fast-Echtzeit auf dem ersten Anzeigebildschirm angezeigt wird, **dadurch gekennzeichnet, dass** der medizinische Kiosk ein modulares Design aufweist, wobei ein Innenraum durch eine oder mehrere Seitenwände, Frontplatten, Rückplatten und Bodenplatten definiert ist, und wobei der Innenraum derart bemessen und konfiguriert ist, dass ein Zugang in den Innenraum gestattet wird, und jede der einen oder mehreren Seitenwände, Frontplatten, Rückplatten und Bodenplatten eine Länge und eine Breite definiert, die durch eine Standardtür passt; und der Innenraum mit einem oder mehreren medizinischen Fächern versehen ist, wobei mindestens eines der Fächer mindestens ein medizinisches Gerät beinhaltet, das zur Verwendung durch den Benutzer konfiguriert ist, um Informationen über den Benutzer an den medizinischen Dienstleister bereitzustellen, um dem medizinischen Dienstleister das Bereitstellen von medizinischer Echtzeit- oder Fast-Echtzeit-Beratung an den Benutzer, während sich der Benutzer in dem Kiosk (100) befindet, zu ermöglichen; und wobei das eine oder die mehreren medizinische/n Fach/Fächer (ein) ferngesteuert verriegelbare/s medizinische/s Fach/Fächer ist/sind, wobei der medizinische Dienstleister das medizinische Fach aus der Ferne und selektiv verriegeln oder entriegeln kann.

2. Medizinischer Kiosk nach Anspruch 1, wobei die Kioskstruktur einen Innenraum beinhaltet, wobei der Innenraum die Frontplatte, eine Rückplatte und erste und zweite Seitenplatten beinhaltet, wobei sich der erste Anzeigebildschirm, das Informationseingabegerät, die Kamera und die mehreren medizinischen Geräte in dem Innenraum befinden, wobei der Innenraum eine Zugangstür aufweist, um einem Benutzer das Betreten und Verlassen des Innenraumes zu ermöglichen, wobei die Zugangstür zwischen einer offenen und einer geschlossenen Position beweglich ist, wobei die Zugangstür dem Benutzer in der geschlossenen Position Privatsphäre bietet, wenn er/sie sich in dem Innenraum befindet.

3. Medizinischer Kiosk nach Anspruch 1 oder 2, wobei die Frontplatte einen Schreibtisch (202) beinhaltet, wobei der erste Anzeigebildschirm in einem Abstand über dem Schreibtisch angebracht ist.

4. Medizinischer Kiosk nach einem der Ansprüche 1-3, wobei das Informationseingabegerät auf dem Schreibtisch und unterhalb des ersten Anzeigebildschirms positioniert ist.

5. Medizinischer Kiosk nach einem der Ansprüche 1-4, wobei das Informationseingabegerät eine Tastatur oder einen Touchscreen beinhaltet.

6. Medizinischer Kiosk nach einem der Ansprüche 2-5, wobei die Frontplatte wahlweise mindestens eines der medizinischen Fächer auf jeder Seite des ersten Anzeigebildschirms befindlich aufweist.

7. Medizinischer Kiosk nach Anspruch 6, wobei mindestens eines der medizinischen Fächer ein verriegelbares medizinisches Fach ist, wobei das verriegelbare medizinische Fach zum Verhindern eines Zugriffs durch den Benutzer auf das medizinische Gerät in dem verriegelbaren medizinischen Fach konfiguriert ist, wenn sich eine Tür zu dem verriegelbaren medizinischen Fach in einer verriegelten Position befindet, wobei das ferngesteuert verriegelbare medizinische Fach eine bewegliche und verriegelbare Tür aufweist, die einen Zugriff auf das mindestens eine medizinische Gerät in dem ferngesteuert verriegelbaren medizinischen Fach einschränkt, wobei die Tür zwischen einer geschlossenen Position und einer offenen Position beweglich ist, wobei die geschlossene Position der Tür einen Zugriff auf das medizinische Gerät in dem ferngesteuert verriegelbaren medizinischen Fach einschränkt, wobei die offene Position einen Zugriff auf das medizinische Gerät in dem ferngesteuert verriegelbaren medizinischen Fach gestattet, wobei die Tür an dem ferngesteuert verriegelbaren medizinischen Fach durch den medizinischen Dienstleister selektiv entriegelbar ist, damit sich die Tür aus der geschlossenen Position in die offene Position bewegt.

8. Medizinischer Kiosk nach einem der Ansprüche 1-7, der eine Registrierstation beinhaltet, die außerhalb des Innenraumes positioniert ist, wobei die Registrierstation ein Benutzereingabesystem aufweist, das dem Benutzer das Eingeben von Informationen über den Benutzer vor der Echtzeit- oder Fast-Echtzeit-Telekonferenz mit einem medizinischen Dienstleister ermöglicht, wobei das Benutzereingabesystem wahlweise eine oder mehrere Komponenten beinhaltet, die ausgewählt sind aus der Gruppe bestehend aus einem Tastenfeld zur Identifikation, einem Tastenfeld zur Dateneingabe, einer Tastatur zur Identifikation, einer Tastatur zur Dateneingabe, einem Touchscreen zur Identifikation, einem Touchscreen zur Dateneingabe, einem Mikrofon und Spracherkennungssoftware zur Identifikation, einem Mikrofon und Spracherkennungssoftware zur Dateneingabe, einem Fingerabdruckscanner zur Identifikation, einem Fingerabdruckscanner zur Dateneingabe, einem Netzhautscanner zur Identifikation, einem Netzhautscanner zur Dateneingabe, einem Kartenlesegerät und einem Lesegerät/Scanner für intelligente Geräte.

9. Medizinischer Kiosk nach einem der Ansprüche 1-8, der eine Bodenplatte beinhaltet, wobei der medizinische Kiosk ein oder mehrere Zubehörteile aufweist, die zumindest teilweise auf oder in der Bodenplatte positioniert sind, wobei die Zubehörteile ausgewählt sind aus der Gruppe bestehend aus einer Bank und einer Waage.

10. Medizinischer Kiosk nach einem der Ansprüche 1-9, der ein Reinigungs- oder Desinfektionssystem (220) zum Reinigen oder Desinfizieren zumindest eines Teils des Innenraumes beinhaltet, wobei das Reinigungs- oder Desinfektionssystem (220) ein System aufweist, das ausgewählt ist aus der Gruppe bestehend aus einem UV-System und einem Sprühnebelsystem.

11. Medizinischer Kiosk nach einem der Ansprüche 1-10, der einen Betreuerschreibtisch (200) aufweist, der außerhalb des Innenraumes positioniert ist.

12. Medizinischer Kiosk nach einem der Ansprüche 1-11, der eine Deckenplatte (210) und ein Lüftersystem (212), das sich auf der Deckenplatte (210) befindet, beinhaltet, wobei das Lüftersystem (212) ausgelegt ist, Luft in den Innenraum zu ziehen, Luft aus dem Innenraum zu ziehen oder Kombinationen davon, wobei das Lüftersystem ein Filtersystem beinhaltet.

13. Medizinischer Kiosk nach einem der Ansprüche 1-12, wobei sich die Kamera an einer Position befindet, die ausgewählt ist aus der Gruppe bestehend aus der Frontplatte (140, 150) oder dem ersten Anzeigebildschirm (340).

14. Medizinischer Kiosk nach einem der Ansprüche 1-13, wobei die Kioskstruktur einen Drucker beinhaltet.

15. Medizinischer Kiosk nach einem der Ansprüche 1-14, der einen Anzeigebildschirm aufweist, der sich außerhalb einer inneren Einfassung des medizinischen Kiosks befindet, der zum Bereitstellen von einer oder mehreren Arten von Informationen verwendet werden kann, die ausgewählt sind aus der Gruppe bestehend aus Werbeinformationen, Informationen über den medizinischen Kiosk, Informationen über eine Wartezeit für den medizinischen Kiosk, Informationen bezüglich der Reihenfolge von Benutzern, die darauf warten, den medizinischen Kiosk zu nutzen, Informationen darüber, ob ein medizinischer Kiosk verfügbar oder gerade belegt ist, Kabel-TV-Shows, Satelliten-TV-Shows, lokal übertragene TV-Shows, Dauerwerbesendungen, medizinischen Programmen, DVD-Materialien, Blu-ray-Materialien, Anzeigeprogrammen, You-Tube-Programmen und Bildern, wobei die Anzeige konfiguriert ist, nur Informationen anzuzeigen und keine Dateneingabe von dem Benutzer zuzulassen.

## Revendications

1. Kiosque médical (100) conçu pour fournir des services de télémédecine à un utilisateur, ledit kiosque médical ayant une structure de kiosque qui comprend un panneau avant (140, 150), une pluralité de dispositifs médicaux et un système de vidéoconférence d'utilisateur, ledit système de vidéoconférence d'utilisateur étant conçu pour permettre à l'utilisateur d'avoir une téléconférence en temps réel ou en temps quasi réel avec un prestataire médical situé à distance dudit kiosque médical (100), ledit système de visioconférence d'utilisateur comprend un premier écran d'affichage (340), un dispositif de saisie d'informations, une caméra, et un système informatique et un logiciel de vidéoconférence, ledit premier écran d'affichage étant configuré pour afficher ledit fournisseur médical pendant une téléconférence entre ledit utilisateur et ledit fournisseur médical, ledit dispositif d'entrée d'informations étant configuré pour permettre audit utilisateur d'entrer des informations, ledit premier écran d'affichage (340) et ledit dispositif d'entrée d'informations étant positionnés dans ledit kiosque de telle sorte que ledit utilisateur puisse visualiser simultanément ledit premier écran d'affichage (340) et ledit dispositif d'entrée d'informations, ledit système informatique et logiciel de vidéoconférence comprenant un matériel électronique et un logiciel pour former une connexion vidéo entre ledit kiosque médical (100) et le prestataire médical situé à distance de sorte que le prestataire médical situé à distance soit affiché en temps réel ou en temps quasi réel sur ledit premier écran d'affichage, **caractérisé par** - le kiosque médical ayant une conception modulaire dans laquelle une chambre intérieure est définie par un ou plusieurs parois latérales, panneaux avant, panneaux arrière et panneaux de plancher, et dans lequel ladite chambre intérieure est dimensionnée et configuré pour permettre l'accès à l'intérieur de la chambre intérieure, et chacun desdits un ou plusieurs flancs, panneaux avant, panneaux arrière et panneaux de plancher définit une longueur et une largeur pouvant s'adapter à travers une porte standard; et la chambre intérieure est pourvue d'un ou plusieurs compartiments médicaux, dans lequel au moins l'un desdits compartiments comprend au moins un dispositif médical configuré pour être utilisé par l'utilisateur pour fournir des informations au fournisseur médical sur l'utilisateur afin de permettre au fournisseur médical de fournir des conseils médicaux en temps réel ou en temps quasi réel à l'utilisateur pendant que l'utilisateur se trouve dans ledit kiosque (100); et dans lequel un ou plusieurs compartiments médicaux est un compartiment médical verrouillable commandé à distance, dans lequel ledit fournisseur médical peut verrouiller ou déverrouiller à distance et sélectivement le compartiment médical.

2. Kiosque médical selon la revendication 1, dans lequel la structure du kiosque comprend une chambre intérieure, ladite chambre intérieure comprend ledit panneau avant, un panneau arrière et des premier et deuxième panneaux latéraux, ledit premier écran d'affichage, ledit dispositif d'entrée d'informations, ladite caméra, et ladite pluralité de dispositifs médicaux situés dans ladite chambre intérieure, ladite chambre intérieure comprenant une porte d'accès pour permettre à un utilisateur d'entrer et de sortir de ladite chambre intérieure, ladite porte d'accès pouvant se déplacer entre une position ouverte et fermée, ladite porte d'accès dans ladite position fermée assurant l'intimité dudit utilisateur lorsqu'il se trouve dans ladite chambre intérieure.

3. Kiosque médical selon la revendication 1 ou 2, dans lequel ledit panneau avant comprend un bureau (202), ledit premier écran d'affichage étant espacé au-dessus dudit bureau.

4. Kiosque médical selon l'une quelconque des revendications 1 à 3, dans lequel ledit dispositif d'entrée d'informations est positionné sur ledit bureau et en dessous dudit premier écran d'affichage.

5. Kiosque médical tel que défini dans l'une quelconque des revendications 1 à 4, dans lequel ledit dispositif d'entrée d'informations comprend un clavier ou un écran tactile.

6. Kiosque médical tel que défini dans l'une quelconque des revendications 2 à 5, dans lequel ledit panneau avant comprend facultativement au moins un desdits compartiments médicaux situés de chaque côté dudit premier écran d'affichage.

7. Kiosque médical selon la revendication 6, dans lequel au moins l'un desdits compartiments médicaux est un compartiment médical verrouillable, ledit compartiment médical verrouillable étant configuré pour empêcher l'accès dudit utilisateur audit dispositif médical dans ledit compartiment médical verrouillable lorsqu'une porte sur ledit le compartiment médical verrouillable est dans une position verrouillée, ledit compartiment médical verrouillable commandé à distance comprend une porte mobile et verrouillable qui limite l'accès audit au moins un dispositif médical dans ledit compartiment médical verrouillable commandé à distance, ladite porte mobile entre une position fermée et une position ouverte , ladite position fermée de ladite porte limitant l'accès audit dispositif médical dans ledit compartiment médical verrouillable télécommandé, ladite position ouverte donnant accès audit dispositif médical dans un compartiment médical verrouillable télécommandé, ladite porte sur ledit compartiment médical verrouillable télécommandé est sélectivement déverrouillable par ledit fournisseur de soins médicaux pour faire passer ladite porte de ladite position fermée à ladite position ouverte.

8. Kiosque médical tel que défini dans une quelconque des revendications 1 à 7, comprenant un poste d'enregistrement positionné à l'extérieur de ladite chambre intérieure, ledit poste d'enregistrement comprenant un système d'entrée d'utilisateur qui permet à l'utilisateur d'entrer des informations sur l'utilisateur avant d'avoir ladite téléconférence en temps réel ou en temps quasi réel avec un fournisseur de soins médicaux, ledit système d'entrée d'utilisateur comprenant facultativement un ou plusieurs composants sélectionnés dans le groupe comprenant un clavier pour l'identification, un clavier pour la saisie des données, un clavier pour l'identification, un clavier pour la saisie des données, un écran tactile pour l'identification, un écran tactile pour la saisie des données, un microphone et un logiciel de reconnaissance vocale pour l'identification, un microphone et un logiciel de reconnaissance vocale pour la saisie des données, un scanner d'empreintes digitales pour l'identification, scanner d'empreintes digitales pour la saisie des données, un scanner de rétine pour l'identification, un scanner de rétine pour la saisie des données, un lecteur de carte et un lecteur / scanner de périphérique intelligent.

9. Kiosque médical tel que défini dans une quelconque des revendications 1 à 8, comprenant un panneau de plancher, ledit kiosque médical comprenant un ou plusieurs accessoires positionnés au moins partiellement sur ou dans ledit panneau de plancher, lesdits accessoires étant choisis dans le groupe constitué d'un banc et une échelle.

10. Kiosque médical tel que défini dans une quelconque des revendications 1 à 9, comprenant un système de nettoyage ou de désinfection (220), pour nettoyer ou désinfecter au moins une partie de ladite chambre intérieure, ledit système de nettoyage ou de désinfection (220) comprenant un système choisi dans le groupe composé d'un système UV et d'un système de brouillard.

11. Kiosque médical tel que défini dans une quelconque des revendications 1 à 10, comprenant un bureau de préposé (200) positionné à l'extérieur de ladite chambre intérieure.

12. Kiosque médical selon l'une quelconque des revendications 1 à 11, comprenant un panneau de plafond (210) et un système de ventilateur (212) situé sur ledit panneau de plafond (210), ledit système de ventilateur (212) étant conçu pour aspirer de l'air dans ledit chambre intérieure, aspirer de l'air hors de ladite chambre intérieure ou des combinaisons de celles-ci, ledit système de ventilation incluant un système de filtrage.

13. Kiosque médical tel que défini dans une quelconque des revendications 1 à 12, dans lequel ladite caméra est située dans une position sélectionnée dans le groupe constitué dudit panneau avant (140, 150) ou dudit premier écran d'affichage (340).

14. Kiosque médical tel que défini dans une quelconque des revendications 1 à 13, dans lequel ladite structure de kiosque comprend une imprimante.

15. Kiosque médical tel que défini dans l'une quelconque des revendications 1 à 14, comprenant un écran d'affichage situé à l'extérieur d'une enceinte interne dudit kiosque médical qui peut être utilisé pour fournir un ou plusieurs types d'informations sélectionnés dans le groupe constitué d'informations publicitaires, informations sur le kiosque médical, informations sur le temps d'attente pour le kiosque médical, informations sur l'ordre des utilisateurs attendant d'utiliser le kiosque médical, informations sur la disponibilité ou l'utilisation d'un kiosque médical, émissions de télévision par câble, émissions de télévision par satellite, émissions de télévision locales, infopublicités, programmes médicaux, DVD, matériels Blu-ray, programmes d'affichage, programmes YouTube et images, ledit écran étant configuré pour afficher uniquement des informations et pour ne pas accepter la saisie de données de l'utilisateur.
